# EUROPEAN PATENT APPLICATION

(11) **EP 4 265 264 A1**
(43) Date of publication of application: **25.10.2023**
(21) Application number: 22382362.6
(22) Date of filing: 19.04.2022
(51) Int. Cl.: A61K 35/76, A61P 31/04, C12N 7/00, A61K 38/16

(54) **BACTERIOPHAGES SUITABLE FOR TREATING A BACTERIAL INFECTION CAUSED BY KLEBSIELLA PNEUMONIAE**

(71) Applicant: Fundacion Instituto De Investigacion Sanitaria Fundacion Jimenez Diaz, 28040 Madrid (ES); Universidad Complutense De Madrid, 28040 Madrid (ES)
(72) Inventor: GARCÍA QUINTANILLA, Meritxell de Jesús, 28040 Madrid (ES); ESTEBAN MORENO, Jaime, 28040 Madrid (ES); GADEA GIRONÉS, Ignacio, 28040 Madrid (ES); FERNÁNDEZ ROBLAS, Ricardo, 28040 Madrid (ES); AGUILERA CORREA, John Jairo, 28040 Madrid (ES); SENHAJI KACHA, Abrar, 28040 Madrid (ES)
(74) Representative: Hoffmann Eitle

(57) **Abstract**

The present invention refers to a bacteriophage deposited at the German Collection of Microorganisms and Cell Cultures GmbH (Leibniz Institute) (DSMZ)with deposit number DSM34222, and to a bacteriophage characterized by comprising a nucleic acid consisting of SEQ ID NO: 1 In a preferred embodiment, the bacteriophages are used for treating bacterial infections caused by *Klebsiella pneumoniae.*

## Description

### FIELD OF THE INVENTION

The present invention refers to the medial field. Particularly, the present invention refers to a bacteriophage (F13) deposited at the German Collection of Microorganisms and Cell Cultures GmbH (Leibniz Institute) (DSMZ) with deposit number DSM 34222 on 17^{th} March 2022 and to the bacteriophage F12A characterized by comprising a nucleic acid consisting of SEQ ID NO: 1. In a preferred embodiment, the bacteriophage/s are used for treating bacterial infections caused by *Klebsiella pneumoniae.*

### STATE OF THE ART

The increase of multiresistance in bacteria is becoming a major public health problem, especially in hospital settings with increased dissemination of resistant strains due to the globalized world. The most important bacteria in this regard are carbapenem-resistant *Enterobacteriaceae* (CRE), methicillin-resistant *Staphylococcus aureus* (MRSA), extended-spectrum betalactamase (ESBL) producing *Enterobacteriaceae,* vancomycin-resistant Enterococci (VRE), multidrug-resistant (MDR) *Pseudomonas aeruginosa,* and MDR *Acinetobacter* sp. According to the latest reports from the Center for Disease Control and prevention (CDC) one out of four healthcare-associated infections will be caused by one of the above-mentioned bacteria. In 2019, the European CDC reported that multiresistance is mainly worrisome in *Acinetobacter baumannii* (43.6%), *Klebsiella pneumoniae* (15.6%), *Escherichia coli* (7.3%) and *P*. *aeruginosa* (3.9%). Moreover, the WHO has published the list of pathogens with critical priority in research, development and innovation of new antimicrobial therapies. This list includes carbapenem-resistant *A. baumannii,* carbapenem-resistant *P. aeruginosa* and carbapenem-resistant and ESBL-producing *Enterobacteriaceae.* The worldwide dissemination of these pathogens, together with the appearance of new forms of resistance (including structures like biofilms) has led to the development of the "post-antibiotic era" concept.

The shortage of new antibiotics in the market leads to find alternative therapies, such as phage therapy, trying to eliminate these MDR pathogens and avoid a global medical crisis. Bacteriophages are viruses that can infect and multiply inside bacteria. They are biological systems ubiquitous in nature and very diverse from the genetic point of view; they were discovered by Felix d'Herelle in 1917. However, the first suspicions of the existence of microbes antagonistic to some bacteria were made by the British bacteriologist Frederick Twort. Phages are classified as virulent or temperate depending on the biological cycle they perform, lytic or lysogenic, respectively. Lysins are enzymes encoded by phages responsible for the bacterial cell wall lysis at the end of the lytic cycle and are interesting for their ability to disrupt biofilms. Virulent phages are the most desirable for therapeutic use against bacterial infections. The first investigations were carried out analyzing the possible role of these viruses in medicine. However, the discovery of penicillin by Alexander Fleming in 1928 and the subsequent development of clinically successful antibiotics in the following decades of the 20th century brought these lines of research to a standstill. Phage therapy has been poorly investigated.

A PubMed query in April 2021 with the terminus "phage therapy" revealed 1664 publications, most of them (96.69%) from the last 10 years. During decades, this field has suffered from a significant lack of data about many aspects such as limited knowledge about tolerance, immune response, pharmacokinetics, pharmacodynamics, and proper experiments using animal infection models. Recently, phage therapy research has been reintroduced in Western countries such as France, Belgium, the Netherlands, the United Kingdom, Switzerland, and the USA, among others. Moreover, in 2014, the US National Institute of Allergy and Infectious Diseases included phage therapy as one of seven strategies to tackle antibiotic resistance. Some Eastern countries have traditionally used phage therapy under compassionate use in patients (after failure of conventional treatments) with two main Centers providing bacteriophages: The Eliava Institute in Tbilisi (Georgia), and the Ludwik Hirszfeld Institute of Immunology and Experimental Therapy in Wroclaw (Poland). Currently, the process and conditions of compassionate use are regulated by different agencies depending on the country: The Food and Drug Administration (FDA) in the USA, the Therapeutic Goods Administration (TGA) in Australia, or the European Medicines Agency (EMA) in the European Union with additional regional regulations.

*K. pneumoniae* is a Gram-negative bacillus that belongs to the Enterobacteriaceae family and is an opportunistic pathogen responsible for a wide range of nosocomial infections, including urinary tract infections (UTIs), respiratory tract infections, blood-stream-associated infections, surgical-site infections, and also is responsible for liver abscesses in community acquired infections. Importantly, the intestine of patients acts as a reservoir for *Klebsiella pneumoniae* within a hospital. This pathogen causes severe morbidity and mortality in ICUs, premature ICUs and medical, pediatric and surgical wards. *K. pneumoniae* strains are intrinsically resistant to penicillins and can acquire resistance to third and fourth-generation cephalosporins. Third-generation cephalosporins have been used as monotherapy and in combination with aminoglycosides against *K. pneumoniae* infections. Fosfomycin is mainly used in UTIs. This practice accelerated the emergence of fluoroquinolone-resistant and ESBL-producing *K*. *pneumoniae* strains which are treated with carbapenems. Carbapenem use has also increased the appearance of CRE bacteria which are resistant to carbapenems, penicillins, cephalosporins, fluoroquinolones, and aminoglycosides. Carbapenemases are a class of enzymes that can be transferred rapidly through conjugative plasmids among the Enterobacteriaceae family. Treatment options against carbapenem-resistant *K*. *pneumoniae* are limited to colistin and tigecycline. Unfortunately, resistance to these antibiotics has also been described. Moreover, carbapenem-resistant *K*. *pneumoniae* is associated with 50% of mortality rates in Europe. A systematic review and meta-analysis of mortality concluded that patients infected with carbapenem-resistant *K. pneumoniae* strains exhibited higher mortality rates (42.14%) than those infected with carbapenem-sensitive strains (21.16%), especially in association with bloodstream infection, ICU admission, and solid organ transplantation.

So, there is an unmet clinical need of finding reliable therapeutic strategies, particularly reliable bacteriophage candidates, able to inhibit the growth of clinical strains of *K. pneumoniae* (including carbapenem resistant strains), both in liquid and in mature biofilms. The present invention is thus focused on solving this problem and new strategy is herein provided.

### DESCRIPTION OF THE INVENTION

### Brief description of the invention

The present invention refers to bacteriophages named as F13 and F12A in the present invention. F13 has been deposited at the German Collection of Microorganisms and Cell Cultures GmbH (Leibniz Institute) (DSMZ) with deposit number DSM34222 on 17^{th} March 2022. F12A is characterized by comprising a nucleic acid consisting of SEQ ID NO: 1. These bacteriophages are characterized by their specificity for *Klebsiella pneumoniae* strains.

It is of utmost importance to consider that, according the results provided in the present invention (see **Example 2** of the present invention), the bacteriophage/s of the invention are able to inhibit the growth of clinical strainsof *K. pneumoniae* (including carbapenem resistant strains), both in liquid and in mature biofilms. Growth curves showed that the phage exhibited antimicrobial effect alone.

So, the first embodiment of the present invention refers to a bacteriophage (hereinafter *bacteriophage of the invention)* deposited at the German Collection of Microorganisms and Cell Cultures GmbH (DSMZ) with deposit number DSM34222 on 17^{th} March 2022, or characterized by comprising a nucleic acid consisting of SEQ ID NO: 1, or a bacteriophage comprising a nucleic acid having at least 95% of identity with the nucleic acid of the bacteriophage DSM 34222 or the SEQ ID NO: 1.

The SEQ ID NO: 1 of bacteriophage F12A is as follows:

GCGTATGAAGCCTACACCGTAGCATCTGATGGCGCTGAACTCGTTGCAGTTCGTCGTGCTGGCAATGCTGCAA
TGCCTGCGGCGTAAGGAGCCGAACAATGGAAAAGATTATCTTTACGAAAGGCCTGATCACCAACTCGCAGGT
GGTTAGAGAGCAGTGGCGCCACCTTACCGCTGACCGCAAGGTTTTCATCAATGGTGAAAACGCTCTGGCGAAA
GAATACGGCGTGAACGCCACCGCACTGGTAACGAAAGACTACTGGCGCGAAGTGGACGACGTGACCACCCGT
GTATTCCGCAACGAGTCCGGCATGGACATGATGGCCGACCTGATGACGCTGGCGACCAACATCAACATCGGTA
AGACCGTGGCAATTAGCCGCATGGCCTCCGATGCTGGCAAGGTTGTGCGCACCATCTCCGGTCAGGAGCCTGA
AGACCTGGATAAAACCCGCTATTCCTACAGCGGCGATGTAATCCCGATTTTCAAAACCGGTTATGGCCGCGAG
TGGCGTGAGCTGCTTGGTATGCAGTCTGAAGGTTTCGACCCGCTGATTGATGACCAGGAAAGCACCACCTTCA
ACCTGCGCGCAGACATGGCGGATTATCTGCTGGTCGGCGATGCAAGCCTGAATGTGAACGGCGTTTATACTGC
TTACGGCATCACCAACCACCCGAACACCGCGCAGCTCAACCTGAGCACCTCTGGTACTGGCGCGCTGAACATA
GACCTGCAAACGGCAACTCCTGATGAAATCGTTGAGTTCTTCAACCAGGATTTCCAGGCGGTTCTGGATGCAC
AGAACGTGTTCGAGCCGGTTACTCTGTGGGTTTCTCCGTCTGTGCGCCGTTCCTTCAGCCGCCCGTACTCCAAC
GCGGCAGGCTTCAAAGGCGGCACCATTGAGGATTATATCCTCGCATTCGGCAAAACCGGCAATGTTGGCCGCA
TCGCGTCTATCGGCACCAACTTCAAGCTGACCGGAAACCATTTCGTCGGCTATGTGAAGAATGCGCTGTATAT
CCGCCCTCGCGTCGCTCAGCCGGTATCTACCTATGCAGAGCCGCGTACCACGCCGCACGCTAACTTTAACTTCC
TGACCTGGGCTGCTATGGGCCTGCAAATCCGTAAGGATTTCAATGGTCGAAGCAAAGTTTTCAACGGCTACGG
CACGCAAACCGCGCTGTAAAAATAAAGGGGCGAAAGCCCCTTTTAACTATCAGAGGTTATCATGGCTAAATAC
GAAGTTATCGCCAGCGGAATCTTCGTCAAGGATAAAGATGGTCGTCTGCGGGAGCTTGCCATTGGCGATATCA
TTGACGAATCAAGCCCACACATTGAGTCAAAACTTCGCCCGGTTAGTGAGAAAATTCTGGAAGTTGCAACTCC
GCAAGAATCGCAGCCAAAGACAAAGAAAACCAAGTAAAATAAACCCGCAAACAAGCGGGTTTTTTATTGGGG
TTTATCATGGCTGTCAGGTACGAAATAAACACAACTCCGGCTGACGGAGAGGTTTTGCTTAGCGAAACCATGT
CAGCTGACTGGACTGCATTGCAGGTACAGATTGTTCCATTAAACTCTTCCGGCGGCTATGAGCCATTAACAAG
CGGCTCTGTTTCCGTAATGGTATCGCCGTTTGCATCTGGTGACTTCTGGATTGACGTAAACAATAATAATTATT
ATGGCGTGGCGCTGCGACTGAAGGCGATCAAGTCACAACTCCCTGCTGGCGTTGCATCTCTGAAAGTCCTGGT
ATGGCGATCTGACACCTCAGTACCATCAAGTCAGGTTGTCGCGCAGTCATATGCGGAGCTTGCCAATAAGCAA
GGAAAGCTATTCACCGCATCGCGTCGCGTGACTGATGTCGCTGGCGGGGCTAACCTTGACAGCATTTTTATTA
CCGGCTCAAAACAGGTTGTGTTTAATCAGCGCATCATTGGCCACACCGGGAAAGGTGTTGTGGCGTCAATCTA
CCGTGAAGCCGTAGCCACTGGCGGCACCGCTGCGGAGATTAATAACCCAAATGACATCGCGCCGCATACTGC
AACAGCGCAGCTTCTGACTGGCTCTACGATTACAAGTATTGGGCAGCTGACGGTGGCGGCTACGTATAGTGAA
GGGAACGCATCCAATCAGGGTCAGGGCAATTCGCAGGCAAAACTCGGTGAACAGGTCATCATGGCGCCTAAT
ACCACCTATCTGTTGCGCATCACGTCCCTGGATACTGCGGCGCAAAATATCAATGCTTACGTTTAGTGGTTTGA

GGATGACGCTTATCTTCCGTGATAAAAGAAAAGCCCCGATAGGGGATTTTTTCTACTTGACTAGTGATGCGGC
GTATTCGATAAGGTAGAGCTTTGGTGCAAGCCAGATTTTTAGCCAGTCGAAATTATTAAATATGATAACGATA
CAGATGATTGAACCTATACCAGTAAAAACCAATGCCACAAGAACAACTTCACCATCATCACCTTTATTCCATG
CAAAAATCATTAGATAAATACATGCAATAATAGCCATTACGCAAATGATTTGAATGCCTGCTGAAGATACAGC
ATTCCACATCAACAGTTGCTGAATAACATCAGGAATCTGTGCCTGACTAAATGACACCGCAGAATCAATGCCA
TCACTTGCCTTCTTCAGAAGGTCTTCCAGAATTTTTTGTGCTTGTTCGTTCATAACTAAATCCTCACTTATGGTC
GCGGAGATTGACGAGGCCATCAATCAGCCGCTGAATTGAGGCGTTGTCTTTAAATGTGATATAAATTTCCGCC
TCATCAATATCATCAACTTCATCGCCCGGCACTCCACGATTTATGCTATTCGCAATGGCTATTAACTTTTCACC
ATGGAAAGAGCCAAATTTAATGCCAACGTATCCATCGCCAAGGCGGGCAACTTTTGAGCCATCATTCTGAGTA
AAAACGCCATTCTTTTTCATCACTAAATCCTCATTGGCATAACTGTTATTTTTTGTTCTTGATGTAATGCTGTTT
TGCGATATATAGCATCTCATCAAATGATTTCCCGGTAACGCTTCGGCATTGTCGATAGTGTTGTAACGCAGCCT
CTATGGCCGCGTTGTCGATTCCTGGCAGTTTATCGCGCAGGTTTTTCTCTATGAATTGTTCTGGGTTCATCCTAA
AATCCTTTTTTTGGCTATTTCAAAATACACATCATCTTTTTCAATGCCGATAAATCGACGGCCGATGTTCATGC
AGGCAACTCCGGTTGTGCCGCTGCCCATAGTGAAATCGAGAACCTTTTCACCTTCGTTGGTGTACGTCTTAATT
AGGTATTCCATCAGGGCGACAGGCTTTTGTGTCGGGTGAACCGTTTTGTTTTCGCTTTGGAAATTCAATTGGGT
ATGTGGGTACCCTGAGAACTCCTGTTTATATATTGGCTGATGCAGCTTATTATTAGGTCCGAGATTCTCACCTT
GACGACCCAAAAACCCAACACCTCCACCCCCATTTTTGACAACCCTATCAACCCTAACAAGCCCTTGCGGGTT
GTAGCGCATTAATCTCTTTGAGCCGTTGGCCACCGCCCCATTCGAGAACACCAAAACAGACTCATGCGCTCCC
ATTGGCTTTAGTTTTGCCCTAATGAAGTCTGTCCGCCTTGTCTTATTCCACACCCAGTCATATTTAAACATTGCC
AGATTGCTGCAAACTAAAATGCTAGTGAATGGTTGAGTGGCAGTCATCACAATAGCGCCGTTTGGCTTAATGA
TGCGCTTCAATTGTTCACACATTGGCTCAAGAGGGATGATGGAGTCCCATTTGCAGGCGGTTGTCCCATATGG
CGGATCGCACAGTACCAAATCAACGCTACCGTCTGGAATAGACTTCATAACTTCCAGGCAATCACCATGAATT
AACTGAACATCTTCATTTATAATCATTACAACTTCTCCAGAATAGCCAGCACTTCATTCAACTCCGCAGATGGA
AGGCGCAAAAATTCTTCTGTCTCCTGTGCTACATGACCCTCAGCAACTACCATATGATCTGCTTCTTTCAGCAA
CTGAATCAGGCGGTCAATCGGCTTAACTTTTTTGGCCTTGAGTGTTTTCGCCGTCACCTTATCTTTGCCCTGCGC
TTTCGCTTCCTCAACGGCAGCATCAATAACATTAACGGCATCATCGCCATGCTCACGTGCGACCGCAACGGCG
TTGGCATAGCTGATTTGCCCTGCATTGATGCGCTGCTTGATGGCATCAGGCACATCGCCAAGCGACAGGTGCA
TCTGCACATCAGAAACTGAGCGACCGACCTTCTTGGCGATTTCTTCATTCGTCCATCCGAACCCTTTCAGGCGC
ACATAAGCCTTTGCACGCTCAAGCGGGTCAAGCTGCTTACCCTGACTGGATGACACCATGAAGGCGATTTTAT
CCGCTTCATCGCCGGCGAAGTCTTTGCACTCAATGCGCGCAATTGGTACATCGCGCTCAATGGCACGCAATGC
GCCAAGGTAACGATGCTGGCCGTCAAGAATCTTAATGCGCTTTCCGTCAGCATCAGGAATAACAGTTAACGCC
GGGATTGGCTGGCCTGATTCCCAGCACTGCGCGAAGTATTCAACGTGCTGCTCATCGGCTTCGCGGATGTTGT
ACCCCGGCTCAAGGTAAAGCTGATCGACAGGAACCTGATAACCTTTGTTGACCACGATTCCGCCGCGAGTTTC
TTTGTCTGAGTAAATTTTTCCGAGAGATGTCATCTTTTCCTCACTTTGATAAACAGATTGCGCTGGCAATGGCG
AATCCGATAACGATTAACGCCAGCTTGATTTTGAACTTATCCCACGCCTTCAGGTCTTCTTTGCGGATTTCGTG
GCGGATCATTGGGATTCTCCGAGTGCTTTTGCAATGGCCGCTTTAGCTTTCGAGGTGTTGACGTAGATGCATGC
GCGAGCTTCTAAATCCAGCAGTGCTTCCAGCAATTCAGGCGCGGCGGCGATGAGACGTGCATTTGCGCGAGTT
TCGTCGGCTTCACGATGATTAACACCGTCATAATCCGGGGACAAAACGTTTGCAACTTTGAATCGTCCGCCCA
ATCGTGTTCCGCGAATTCGAATATATGGTCTGTCAACTTCTTCCGGTTTAATTTCCCACGGCCCCGGCGTACCT
TTGAACTCATTCATCTCCATCACCTCTAACATTTATTGTTGTTTCTACGTCATCACTATAGCGACACCCTCAATC
TACGTCAACACTTTATGATAAAATTAAACCAACAATACTCACCCCGCGCTGTTCCGTCCTGAAAAACGTAAGG
CGGCGAAATTGGATATAGCAATCGGCGTTATCGTCATAGCGTTTTCGCTGGTTCAGGTGTACAGATGCTGGAA
GTTCATCATTCGGAGAATAATTAATGAGAGACGCGCTTCAGCACGCCGCAAACCAGATAATTAGTGGCACTGT
CGGCCAGGTAATCGACAAAGCCGGTTATACATCAATCGGCACTGGTCTTGGCCTGAAGGTGGCAGAGCAGAC
GCCGGTCGCACAATCATACATTGCCTCAATGATCCCCCATTCGATTACCGAGTGGGCAGCGGTAGCCTCTATA

CTTGGCGCGTTGTCACTGGTGGCAAAAAACCTTTTTGAGATGTGGTGGAAGATTCGGGAGAGCAAAAAGAAT
GGCAGCACCGACAGCAAGTGAACTTGTCGCCGCTATGGCGTCAAGAGGCGTAACTATCACCACGGCGGACGC
GACGGGCATCCTGTGTCTGGTGGCGAGCATCACAGAGTGCCTTGAGCTTAATTACCCTGAAGACACATGCAGG
CAGGATGCGATTCTGCTATGGGCATCTATCCTGATCGCCTCAAATACAGCCGGAAGATACATCACCAGCCATC
GGGCGCCATCTGGTGCGTCGCAATCATTTGGCTATGGCAGCAAGCCGTGGATGGCTCTTTACAATCAGATGAA
GCTACTCGATACAGCAGGCTGTACAGGCGACCTTGTGGAAGAGCCTGACGGAAGTGCAAAGCCGTGGTTTCG
AGTTGTTACCGGGAGTAAGTGCAAATGAAAACGTCAACATTAACTCTAAATATCGCAATCCGTAAATGGTGCA
TGCCGCTGCTGGTTATTCTGGTATTGCTGCGCCTTCCTGTTCCGCGCTGGGTTTATACTCTTGAGGTCACACCAT
GTCAGCAATAGCGAGATGGAGCTATACGCAGCCATGCACAATCTGGCGGCTTACTGGCAAGGATAAGTATGG
CAAGCCAACATTCGCCGCGCCAGAATCCATCATGTGTGATTATGGCTTCGATAAGAATCTGACCACCGGCACA
GCTGGCAATGAGATTGCGCAGAAAAACACATTCTGGACGGAATATCAGAATGCGTCTGTTGGCGACTTCATCA
TGCTTGGCACCGTCACAAGTGCTGACCCGCTGGCCGCCGGAGCTGACCAGATTAGAAACGTTGTGAATTACGG
TAATACGTTGGACCGCAATGACCTGCCTGATTTTGCACTGGTAACGGGGTAACGTATGGCCGCCAAAATGCGA
GGTATCCAGCAGGCGATTAAGCGAACCCAACAGATAGTCGGAGAGATTACTGGTGAAAAGGCTATGCGGGCC
ATAAAGAGTGCCAACTTTATTATCCGCACTGAATCAGCCAGCATGACGCCGATTGCCACGTCAACGCTGATAA
ACAGCCAGTATGATACCGTTGAGGTTAATGGCACTCGTATAACTGGCAAGATTGGCTATTCCGCCAATTACGC
CCTGTATGTCCATAATGCACCTGGTACACTGCTTGGCACGAACACGCCGCGCACAGGGAGACTTAAAGGAAA
GGGAAATGTATGGGATAAGAGCGGCGAGCCTAAATTCCTTCTCAAGGCTGGCGAAAACACACGCGAGCTTGT
CGATAAGGTAATCAAAAAAGAGATGACGCTAAAATGAGAGATATGCTTGAGCTTGTTGACCAGTACCTTAGC
GACGCCGGTCTTTATGACGGGTGGACTAATCAGCTTGAGTTCTGGAATGATACCGAAGTTGGCACCGACCGGT
TTATGGTGCTGCAATCCAATGGCGGCACGAACGTAAGCAAAGGACTCGGCGGCGATTACTATTTTTCGCTCTA
TGTTGTCGGCCAGCAGGGTCAGTACAATATCGAGGAAACAAAAGCAAAAGCACTTGATGTCATCGCATACAT
CAAGGAACATCCAGTTGATAGTTGCATTGGCATGATTCAGTTGCAGGCGCCGCTTGGCCGCCCTACGCTTACG
ACAGAGCAAAGGTCGGTTTATGAGTTGTTGCTGAGGGTTGTTTTTGGTGAGTAGGCTCGCACTTACCAATAAG
TGCATGTCAACATGATATAATGCGATTGTTTAGCTAAACACAGAGGATTCTAAACATGGCTATTTGTGCAAAT
GATAACGGCATCATCACAGGTCGCCAGTCGCTCATTGAGCTGGCTGATGGCTGCCGGGATGCTGTGCCAGCAG
AGGAAGACTGGAAGTTTTTTGCTCCCATGACCTCAAAAGGCGTCGACTTCAGCCCAAGCACCACTACCTCAGA
GGCTGATGATGGCGATGGCTTTGTCGCAACGCTGGTCACTACCGCAGACCTCACCATTTCCGGTGATTTTGAA
GTTCGCAAGGCTGACAAGGCTGATGAGTATGGCGTGCATAACCTCATCAAATACTTTTTCACCGAAGTAAAAG
ATCGTCGCCAGCCGTCGCTGTGGGTACGCCAGACCACCGGTAATACCGCTGTCGTTGCTTACTGCAACATTAC
CGCCCTGAGCTACGATGGCGGCACCAACGATATCATCACCGGCTCGGTTGAATTTAAGCCTTATGATGGCTCT
ACCGTTGACGTTTCCAGCATCGAAGATTTGACGCTGACTACTGATATCAGCGCAACCAAAAGCGTTGCCACTG
GCGCCACTCTGACGCTCGGTCCGGTGGTTGCGGCTGGCGGTGTAGAGCCTTATACCTATCAGTGGTACAAAGG
CACGTCTCCGATTAGTGGCGCAACCACTGGAACTTTCACCAAAGCAACTGCTGCCGCTGGTGATGCCGGTACG
TATTTCTGCCGCGTGATGGATTCGGCGAGCAGCCCGGATTACGTTGACTCGACTAAGTGCGTAGTCACCGTTA
CCGCATAAAACAAAACCCCCGAAAGGGGTTATTTTTCCTCATGCTTTCCTCGTCTTGTTCTTGATGCCCCAC
AAAATCAGATTAGCCATAAACTCCTAGCGCTTTAAGTAAATGGCAATCGCGTAGTTGATGTCAACGCGCTGGC
CTGCGTAGACGGATATGACATCGGCATCGGATTGGTCAACGTTCATGGTTAGCCTCATATACCGCTCTGGCAA
ATCCGCGCGGTGTTAGTGAACGAAGTTGCTTGGTTCTATGGGATTTGCCACCCAGCCACCTCCAGCCCCAGAA
CATTCCAATATTTACCGGGCCTGGATTTTTCTCTGGCATTACAAACCCGCCACCAGCCCAAATGCAGGTCTTTT
TCGTGTACCCGTCATAAGCTGGCATCTTCGGGTGAAAACTTTCCTCGCTGCCGTCCATGTAACCGCCATACTCA
TACGGGTCGAAGTAGTAATCCGGCTTGCGCCAGAGCGTTGACATCTTGCCCACCGGGTTTTCCACCACCCAGG
GGATTCCGTAGCGCCCGGCGATTCGCTCGACAAGACGAGCGCTATGCAGGGAAGCTAGTCCTTCATGTCCATG
CTTTGCGCCGGATGAGGCGAACTCAGTGCAGTCAGGGAAGGCAAAGATAATCCCTGGGGCTGGAATTCCCAT
AGTCCGCACTACTCCATTAAACAGGTCATCAATCCAGATATTTACATAGTGAATGTTGCTATGCTTAACACGA

ATTGACTCATAATCGCCATGATTCGCGCCATCATAATTAAAGCAGTAGCATTCATGCCCTGCCTCGGCCCACG
GTTTAACCATAATCCCTGAGCCATCAAACATCGACCAGACCACCATCATGCGCACTCATCCAGTTGACGGCGC
AACATACACAGCGCACCATGCGGCATAAACTGCGAAACCATTCCGTCGAATATCTGCCGGTTTAATTTATTAT
CAATTCGCGGCCTAATTGCAGACCAGCACGACCGTATAGCGCGATTAACCTGGCGGCGGTCGAGCATTGCGAG
TCGCGCAGCTAACTCAATGGTAACGAGCGCGTCAAGATACTGCTCGCATGCGTAGCGGCTTTCATTATTCATTC
TTAGAGCCTTATCATCTGGTGTAACGCGTAGCGTAATGGTAAGAATCCATGCACCATTTAGCTGCCTCCTGCCT
ACCAGTTGTTGTACCTATCAGGATAATGCTTACAGTGCCATCTCTTGAGTTGCGCTTTGACTTAACCTCTTGCA
TATCAAGGATGTCGCCTTTTTTGAGTTCGCTGGCAGCAATGTACATCTCTCTTCTTGCCATCACTCACCCTCCTG
CATCATGAGGAAGACAATCATGGCGGCGCGGAGTGGGTTTTTATGGTTCACGCGATGTTTAACATATGCAGGC
TTGAATTGCGCTCGCCACGTTTTTTCGTGCCTGTTCACCACAATACATTCCAGAGAAATGCCATTATTAACAAT
AATCGGCCATGCATCTGCTGGGTTGTTGCAGTAGTCAAAAAATTCATTAGTGCCATTCAAATGGAACACCTGA
ACATCGGGGCGATGATTGTACGGAGTTGTTGTTTGTGACCTGCTGTAAATCAAAGAGGCAACAGCCTTATTAA
TCTCAAAATCACTCATCTTGCTGTAATCAGTGCTCATCTCTTATCCTCCACCACTACGCCGACCTTCGCCAGCA
ACAATATCGCCTTTACCCGAGCCTCTTCATATGTATAACCCTGGTCGATGTACAGGTCGATGTAAAATCTCAAA
TCAGCATCTGTTTCGCTCATCTTATGCGCTCCTTATGGCTTACAGAATCAATCTACGTCAGTTTTGCACCCCCTG
TCAATGGTATAATTACGTCATTGTGAAAACAGGATTTAGACATGAGACAACGCACACCCCTAACAGAAATCGG
AGAGATGCGCATCTCCCTGGCTGACAAGTCTTTTTTCTTCAAACCATCATTTGCGGCGATGAATGAGCTTGGCT
CACCGAAAGAGATTGTCGAGCTGTACGCTACGCTTAATGGCTATGAATACGCAGCCATACTCGGTGCCATTCA
GTCAATGCCATATGGCGCGCAGATTCAGGTGGCAAAAATCCTGTCACGTCCTGCCTATGGCAAGAAAGTGCTC
AGCGCCGCATGCCTCATCATGCAGTCCTGTTGTGATGATGATATCTCGGTGCTCATTGGGTCATGGAAGCCGA
CTCCGCGCGGTGTGAAGTATATTCCTGGATTGATACCAATAGGAGGAACCGGGCATCTCCCTAATGGAAATGC
TGGAATCATTGATATTGCTCGCGCATTAATAGAGCATGGCATCATCGGCAAGTCTCCGCTCAAGGTTCCTCAG
CGCTCGGAAAACCAAAAGCGCACAACCAGTGAACTGAGAATGTCGGATTACATCATCTCAGCTCGCACTCATT
TCGGAATCACCCGCGAGGAAGCGGAATACCTGACCATGACCGAGTATCAGCAAATGATAAAATCAAAATACC
CGGAACCGGAAGGCATGACACGCGAGCAGTATGATGCGTCTTATGAACGGGCCAAGCTGAATAAACAGAAAC
TGAAAGAGAAAGCCGCCAGAAAGGCCGCTAAAGCAAAGGAGCGAAATAATGGCTGAAGAAGTTGGCGGCAT
TGTCTATGAAGTCGGCATGGAAGTGTCCGGCCTTACTGCTGGCGCAAAACAGGCTGAGGACGCATTAAATAAT
ATCGACAAATCAGCGCAGACGTCATCCAAAAGCATGGATAAGCTTGATGGTGCTGCATCATCTTCTGGCAAAG
AGCTTTCAGCGCTGGCAAAGGTGGTCAGCTCAATTGATGCCACCCTGAAAGACATGGCGTCAGCATCAAAAAC
CGCAGCTACATCGGTGGAGGAAACAACCTCAAGCGCTGTAGGTGCAGAGCAGGTTATTGGTGCGTTAAATCA
GCAACTGGCGCAGATGCAGCAGGCGCAGGTTTCCGCAAACGCTACTGGCCTTGCGCTCCAGAACTCAGTTAAT
CAGGTCACTCAGGCTATTCGTGCTCTTGGCGCTCAGTCCACTGAAACCGGCGGATCGATATCTGGTATCGACA
GGATGATTGAGTCTCTTGGGAATCAGATCGCCATTCTTGATGAGCAGGCAGAAAAAGGCGCACGTAGCGCTGC
CATTCTTGCCGCTCAGTTGCGAGCTGGAGACAGTGCCACGGATGCGCAAAAGGCAAAGATAGCCGAGCTAAC
CGGTCGACTCTATGACATGAAAAATGGCACTGAGGCTGCCGGGAAGTCAACGGGTAACTTTAAGAATATCAT
GCAACAGGGCGGCTACCAGATTCAGGACTTCATTGTTCAGGTTCAGGGCGGTCAGTCTGCGCTTGTAGCATTC
AGTCAGCAAGGTTCTCAGCTGGCATCCGTATTTAGTCCTGTTGCCGGTGCGGTGCTGACCATCGCAACCGTTAT
TGCTGGCACGCTCATTGCTTCCCTTGGTGACGGCAAAAACGCCGTTGATGCGCTGAAAGAAGCTATTACCACA
ATGGATAGCGTAGTGTCGGTGTCGTCTTCTGGTGTCGCAGTATACACCGACAAATTCGCTCAGCTTGCCAAGG
TCAATGCCAGCGTAGCAACACTTATGCGCCAGCAGGCGCAGCTTGAGTTGCAGGCAGCGCTTTCAAAAGTATC
AGCTGAAGTCACCAAGGCATCCAGCGATTTTATCGGATTTGGTGATTCGCTCGTATCTTCTCTTGGTGGTGGCT
ATGCCAGTGTCAAGCTGTTCAATGACTACATGTCGCAGCTGAATATCACCACTAACAGCTGGACGGAGGCTAT
TAAGCAGGCATCTGCGGCTGGACAGGCTGGACAGACGTCAATGAATGGCATGATTGCTACCGTTGGTGCGCTG
GCAAGCAAATTCCAGCTTTCAGATCAGCAGGCTTTCGAGTTTGCAAAACAACTGTCAGACATTGCCAAAAATC
CATCAGATGAAAAGCTACGGGCTCTGGTGGTTACGCTACAAAGGGTCGGCGAGGGAACATCAAGCGGCGCTG

TAACGGCGCGGGAATATGCCAAGAGATTGCTGGAAATAGCAACCAGCAGCGCAGATGCCACTCAGCGCTTAC
GGCTTTTAAAGCAAATGACTGATGAGCTTACTGACTCTCAGGATAAGGCGCTTCAGCAGGCTAAGCAGACGCT
TTTCATTGAGCGACAAACTGGCGTTGAAAAACAAAAGGCGCTTGCATGGCGTGATGCAGAGAATCAGGGATT
GAAGGCAGGGACTCAGGCATTCCGTGATTATTATAATGTTCGCCTTCAGACGTATCAGCAGCAGGAGAAAAAT
GCTCAAAGCCTCAAGGATGAACGCAGCGCACAAAGCGCATCTCAATCAGCAGCCAAAAAGGCGGCGACTGAG
CAGGAAAATATTGCTAACAAACTTGAGCAGTTGCGCCAGAAATCCCTTCTTTCAGCCGACTCCACCAGAGAGC
TAAGCCGGGAGCAGTCAATCCTTGCGGCTCAGCAATCACTCGGTAAAGGCGCGACAGAGGAACAAATAAATC
TGGCCGGGCAATATGCAGCAAAAGCGTGGGATAATGCCAATGCGCTGAAGGCGCAAGCTGAAGCTGAGAAGA
AGAGAGTTGAAGCGGTGAAGGGTTTTGCCGCCCTTAAATCTCAGACATCCCCAATGTTTGCCGTTGAAACAAA
TTACCAGAAGGATTTGGCAGCGCTTAATGCTTACGCAGTGGCTTACCCGCAAAAAGTAGCAGAGGTTGAGCAG
ACCAGAGCAGCCATTGAGGAGCAATACCGCCAGCAGCGTCTTGATGCTATGTGGCAGGAGTGGAGCCAGCAG
AATGCCGCCACTCAGGCAGTCGCAGCAGCATTCGAGGCTTTTGGTCAGTCTGCCAGTAACGCGCTGACTGGTG
TCATCACTGGCTCGATGTCGCTCAGTGATGCACTGCGTTCTATTGGCTCCAACATCCTTAACAGCGTCATTAAC
TCATTCGTACAGATGGGTGTTGAGTGGGCTAAATCCGCTATCATGGGTCAGGTTGGAATGACTGCCGCGACAG
CAGCTACTGCCGCACAGGCAGCCGGTATCGCTGCTGCAATGGCTCCTGCTGCTGCAATGACATCTCTTGCAAC
TGGTGGCGCTAACGCCATTCCAGCACAGGCTGGCATCACTGCAACCGTCGGTGTTGCTAAGGCCATGTCGGTA
GCTGGAGCGCTTAAAAATGGAGGCCCTGCGCAGGCTGGCTCAATGTATCGCGTCGGCGAGAACAACCTGCCA
GAGATATTCCAGGCTTCTAATGGTAATCAATACATGATTCCCGGCGATAGCGGGCGAGTTATCAGTAACAAGG
ATATTGGCGGGTCATCAGGTGGCATTGTGATTTATAATAATGTCACCAATAACAGCAGTGGCGCCACAGCAAC
CAGCACGGCGCGAGATAATGGAGATGGCACTGTGACCATTGAGACTATCGTGGCAGACATCGAAAATGGCGG
CCCGATATCGCAGGCCATTACCAGCAACACCACTGCAACCAGAAGGGCAACAGAATAATGGCTATCGCTTAC
CCATCGTGGCTACCGCTGGCGCAACGCGCCAGCAAAAACATGACGACTCAAACGCCGTTCCGCAGCGATCAG
CCTGCTGTTGGCGCGCCTATCTTCCAGAAGCTGACAACTGACGTTGCAGTAACATGGAGCCTGACGTGGATAT
TTACCCTTCGGCAGGAGCGAGCATTTCTTCAATGGCTGAGGAGCCCAAACTATCTCAATAAGTGTAATGAATG
GTTTACAATGGATATCGACCTTGGCGGCAGCGGACTGCAAACGCAGACCCTGCACTTTACCGATTATCCAGTA
CAGACCAGCATTAATGGCGGCCTTGTTACATGGACGGGCAATGTCATCTGCAAGACTCTCAATAACTCAATGG
ATGAGTTTGATGATGTTCTCGTTGAGCTTGATGAGAGATGGTATTCATGGCTGGACGAGGTCGTTAACCGAGA
CTTGCCGGAGTATCCATAATGCCAACATTACGTGAATACCAGTCAAAGCGCCCAAACTGGAAGCTGTATGATA
CGATAACTTTTTATCATTCTTCATTTGGATATGTCCGGCTTGTTGGCAATGAGTTTGAGGATGTCGTTCTTGGCG
GCCAGACCTATCAACCGGTGAGGATGGATGTTACCCGCAGCCAGCAATCGAACACGCCGGTTATCAACGCAA
CGCTGAAGTTTGCGCGGCTGGCAAATGACTTTAAGCAATATCTGAAGCTCTGGACTGGCTCTGGAAGGATTGA
GCCAATCACCGCGCTTTATCAGCGCTTTGATGAAACTGACAAGAACACGCCGCTGAAGCCGTACACACTTTAC
GTTAACGATGTGACGCTTGATCAGTCTGATGTGACTGTCTCCATTTCCATCAAAAACCCCATTAACGGCAACGT
GGCAAAACTTTATGACATCACAGAATTTCCTGGACTGCGCACAGTTTGAGTTGCTTATGCTTGGCAAGCCATAT
ATTGACAGGTGTTGCCATGAGGATGCAGTGGATTGCTGGGGTCTTGTGGTTCTCTTTTATCGCCTTTGTCATGG
GATAAACGTTCACCACGACGATTCATATTCAACGGGTGGTGACTTTGTCACCTGCTTTGATAGCGAGGTTGCAT
TCTGGCTAGAAACCACGACGCCGAAAGTCGGTGACGTAGTAGTTGCATATCGCGGAAGCCATCCGGTACATGT
CGCGCTGTGGTGGGGTCGTGATAAAATACTGCATGCGCGAGAAAAGACGGCGGTCAAAACAGATCGCCTGCG
AACACTCGAAAAACTATCAACAAAATTAAGGTTTCTAGCTTATGCCGGTGATTCACATTCAGAAAATGCCAGG
GGTGCCAAAGGAAACTGGTAATGTGGCGGTTGGTACTAACCTGTGGCGCTGGCTTGAAAGTTCTGGCCTGCCA
ACTGATATCCGCATAGCGCTCAATGGGCGTGTTTTTGGCCCTGATGATGAATTGTCGATATCGTTAAAGCAAA
ACGATATTATTAACATTTACTGCCAGCCTCGCGGCGCCATTGGCGACCTTATCAGCACGATACTCAAGCCTGTA
ACTAAGGTTCTTTCTTTTCTGTTGCCAAAAGCATCAACGCCATCAACCAGCACTGGCTCGACGGTTGAATCACC
CAACAACAGCCTGAAATCGCAAACCAATATTGCGCGAAACGGAGAGGCAAGACCTGACAACTTCGGTCAGAT
AAGGGCATTCCCTGACCTGATTCAGGAATCGCTTTTTGAATACATTGATGACCTGAAATATGTCACTGAGTTCA

TGAATTTTGGCCTCGGTAAATACACCATCTCATCTGTTCGTTATGCGGAAACAAATCTTGGTTCTCTGCCCGGC
GCCACTCATGTCATCTACAATCCAGGTGATGTGATCGGGCAAATTATTGAGCCTTACCAGTTCGACGGGCTTG
ATGGTCAGGAGGTTCCAGGGCTGAGCGAGTCAGAGGACACCCCGATAGAGACAGCGACCACGACATCTGTTA
CCAGTGGTGATTATGCTGGCGGTCAGCTGTTAATGGTCATACCAAAAAACACTGATTTCGATTACTTTATGGG
GCTCTCTTTACCACACTCTGTGTCATTAACAATAAATATTACCTACAACTCGACATCCGGGCCAGTTACTGAAA
ATATCCAGCTTAGTGGCAACATCATTTCAGCTGAGGAAACTGAGACAGGCGTCATTCCTGATATTCAGTATTTC
TATAATTTCACCTTCAATAACCTGACAGGCGCAAATCTTGGCAATCTGACTGGCGCAACCATCAACAACACTT
ATTTCCAGATTGTGGATGATGAGGCGCTCGTTGTTGGCCCATATGTTGGGGCTGTGGAATCAACGCAGGTTTG
GGTTCACGTTCAGTCTGAGCTTGGACCTACCAGTGGCACGGCAGATTATCTGATCAAGGTATGGGCGGTTGAT
GACAATGGGGATGCTATTCCCGGAACCGAGGAGCAGCTCGCAGACAGTATTGACAACCCATTTAATCAGACA
ACAAAAACCTATTATCGCACGTATAAGTTAACTCCTACTTATGGGTTGGCTAAGTATGCCATCAGCATTGAAA
GGACAAACAACTCAAACTCTGGCAACCGCGTAACGTTGCAGGCGGCGCACGCCATCAACATCCGCGAGAATG
TAGTTTATCCTGATGACACCCTTGTTAAAGTCACGGTGAAGGCCACGCTTCAGCCCACATCGGTTACTGAGCG
AAAATATAATGCACTAATCACCCGCTGGACGATTGGATATAACAGAGCAACCGGAGAGGTAGATTACACATT
AACTCCATCAAGAAGTTTTGCTGATGCAGTGCTGCACAACTGGCTTATCACTGCCGGACAACCTGAGAGCACC
ATAGATATTGCAAGGCTCTATGAGATTGCAGATTCCCTGCCAGATGCGCGCCTTGGATATTTTGACTACACGTT
CGATGATGAAGATAAATCAATCGGCGAGCGAATTCAGACTATCTGTGACGCCGCTCGCGTCACAGTATTCTGG
GATGATGGCGTCCTGTCTTTCTCGCGTGATGAGCAAAAATCAACGCCTGAAACGGTATTCAATACCAGGAATA
CGCAGGCAGATGGCTACAAGATGTCATATGATATGACCCTTCCAGGTTCATATGATGGCGTAAGCGTTCAGTA
CCGCGACCCAAACACCAACAAACAGGCTTACGTTTATTATAAAGTTGGGGCATCTGGTATCGAACCGGGAGA
GCCAACTAAACCGAAAAAATTCGACATGCTATACATTCGAAACCTGTATCAGGCAACAGACCGGGCTATGCTT
GAGTGCAACCGTCTCATGTACTCACGTCGCGGAATGGAGATAAAGGCGCTTGCTGATGGCGAGTGGGTAAAC
GTTGGCGATATGATTTCTGTTGTCGACATTTATGATTCTGTACAGCAGACTGGCGTTATCCGATCAAGGTCTGG
AAACGTATTTACCACCAGTGAACAGCTCACGGCAGGAAGCGGCCTGTTTGTGGTCATCACTGGCGCCAATGGG
AATGTGTCAGAACGTCTGGCTTGTACCGTTACTGGATTGAATAAATTCGAGTGCGCATTACCATCTGACTTCGA
GTTAAATATTTTTGACGGTGTTAATGTTCAGTCAGAATCAAGATATGCAATCTCCACGGAGGTTGAGCTTGACT
CAACTTTATGGACAGTCAGCCAGAAAACTCCAGGTACAGATGGCACAGTGTCGCTGACTGTAACCGAGTACA
ACGATGCCATGTACGCCTACACCAACCCTGTTGCGTGATACAATAGGGCAATTAATGATTACGGAGATTGCAG
CCGATGGCTACTACCCCAACTAACAAGCCAATCCCTTCTGAAGACCCGCGTGACCTGAAGTTTAACGCCGGGA
AGATTGATGAAGAGGTTAACGGAAGTTCTGATTACTACACCGACCGATTCGGCGTGCAGCGGCTGACGAATAC
CGGCAGGAATAACCAGTTTCAGACAGCACAAGGTGAGCGTGAGGCAGAGTTTGTTGCGTCACAGGTGGATAA
AGAAGCGCGATTTCAACAATTTCTTTTAAATTCTGGTTATCAGTTTTTGGGCGATTACGAAAATGGTCCGTATA
CAATTGCCGCCAGAAACCAGATTATTCGTTATCAGAACGAGTTCTGGCGTTTAAACGCGGCTACCACCCCACC
TTATACAACTACGGGGATTAACAGCACATCATGGACGGTTGATGTCACTCATTTAGTAAGTGTTGGTGATGCTT
CTTTAAGGCAAAATCTGTCTAGTGATAACGGGTTGGGTTTGATAGGTCAGTGCTCTAGCTTAAACGCCATGAG
AGAAATTGAGTTTTCATCTGCTGGTAAACAAATATTCCTCAAAGAACATACTGCTGGGCAGAATGCTGGTGGT
GGTATTTGGTATTGTCACTCCCTGACGTCTGACAGCTCATACGTCGATGATAATGGGTGCCAGATTATCAACGA
TTATGGACAGGTCATCCGCCGTAAGGATGTTAAAGAATTAACTTCAAGTGACTTTGGATTGAAAGCTGGTGAT
ACCATCGACACTGTCCTCACTAACATGTACAAAGCTTCTCGTACATTTAACATTTATGAAGCAAAAATTGAAA
ACCCCGGCTTTGATAAAGGGTATGTATTACAAGGTGGTCTCCGGTTTTACTGTGGGGATAAGCCTTTTTATATC
CACTCTTATTCCATAAGTACGTTACGAGGACCAAACCTTTGGCATACCGGGAATAATGTCGGAGTTACGTTCA
CCCGTTTCAAAGAAGACGGTACGTCACAACAAGCTTGGTCCGGTGGCGGAATCAAGGGCTTCCGCATTTGGGG
CGCTGCATCTTACCTTGTTCAAGGAAATACCGGTGTGGATGCTACACCAGTACGCCTGTCTGATATGTGGCAA
GGAGAGGCTTGCGACTTGTGGATTACCGGGTATACGGGGAATACTAATGGTGCATCGGTTTCTCTATATAACG
AATTTGCGTGGACGGAAGGATCTCTTGTTAAAAATGTAATGGTTCGCCAATCACTGCGTGGTTTAACATTTCTT

CGCAAGCATGGGACTACAGCTACTGATTCATTTTTCCGACCAGAAGTTGATTTTTCATTTAACGCTGGTGTTCC
TGGGAAATCAACACAAGTCATGGTTGTTGGCGATGGAACTGCCGCTGGTAAATGTCTTGTATACGGCCATGAT
ATTAAGCTTACTCAGTGGATGAGTGCCGGGGCATGGCACGATATCGTCAGGCTTGAGGACTACAGTATTATCG
CGGAGACTGGAGTTATTAAAATAGTTGCAGATGGCTACGGCATATCCAAAACTACTGTACCGGAAACCGAGG
TTGTTCACTACATTAATGTGCGCGGACTCAATGCCCGGTTCCGGAGCAGAGTGGAAAACTGGTCGAATCAGGC
TGGTGGTTGGGGTCTGGATTTCCTGAACATCATCTTCCAGTCCAGTATGTATACTAACGCCATGACGTTTTATG
AGTCGGATTTTGACGCGCTGCCAACCATTAACCCCGTCGGGATGAAGGTGCGGTTTAATGGTACGTTCACTGT
TGCTGAGAGGCAGTCTGGCAAGGTGTACACTCTTAATGGGTTGATTCCGGGGATGACACTGAAAGTCAAGCTG
ACGTCCCGCAACGGTAACGACCTTAATGATGCTGTTGTTCAGGAGTGGAAGGTGTTTGTTCGCAGTACCAACT
TCCCGTGCATAGTAGTCCCTATGTCTGGTAGTGCAAACATTGCTACCACCGATGGGTTGGCTGTCACTAATATC
AGCCCAGTGCAGACTGCTACGTTCCTTAAGACGGTTACGCCTACGCAGGCTAGGAACTTCATCGGTCAGAACT
ACGGACTCACTGTGAAGAACGCGAATGACGATAACAGCATATCCTATGCTGTGAACTCAGGTCGTAATATTCG
CTTTATTCTTCCAGCTAACCCTGGGGCGACTACGACATCCCCGTACTCTGTTGAGATAGAGGTACTTTAAATGG
AAAGCAATGTTGAAAAAATCATACCTGTATTTGCGTTAAAAATTGCAAGTGTAGGCGAGATCACAGATGGTCA
GTGCACGTTAACTTTGGAAAGTGGTCAGGAAGTTTCAGACCCTGTTGTTGTGTCAGAAGAGTACGTGCAAAAA
TACGACCCCAACCTGGCGGCTACTACATAATGTGTGAGAATGGACTCGGATTGTACTCTAATTAAATAACATT
AATTTAATTATCTCAAATGAAAAATAACTTTGACAATAAGCGCATCAATTGCGATGCGCTTTTTACTTAAAAGT
CAAAAAGGAATATCATCGTCAAAATCCATCGGAGGCTCGTTTGCCGGTGGTTTTGACGTGTTTGATGGTTGCT
GAGGTTTACCCCATCCTGTTTGCTGATTGCTTCCTGATTGTTGCCGCTGCTGTGATTTTTGGTTTCCATTATCAC
TTGATTTACCACCAATCATTTGCATAACGCCGTTCATAGGCTGCAAGACGATTTCAGTAGTGTATTTTTCAACA
CCGCTTTGATCTGTCCATTTGCGAGTGCGTAATTTACCCTCTATGTATACCTGAGAGCCTTTGCGCAGGTATTC
GCCAGCCACCTCTGCCAGCTTTCCGAAGATAACAACGCGATGCCATTCAGTTTGCTCTTTCTTCTCACCTGTGG
CCTTGTCATTCCACTGTTCTGATGTTGCCAGAGAAAGATTGCACACAGCGCCGCCAGAAGGCATATATTTAAC
TTCCGGGTCTTGTCCGAGAGTGCCCAAAATGATTACTTTGTTGATTCCGCGAGATGCCATAATTTACCCTTAAA
AGTTTTCGATGTTCTGTTGAGATGTTGATGGCTTCTCTTCGTTTGCAGATGACACGGGCGCATCTGCTTTCTGG
AGTTTTGCCGGGTTGAAATCGTCCTGCGGTGTAATAGTTACCGCTGCTGGAGATTCATCATTAATGAATGATTC
AATGCGCTCATACTCTTCTGCTGATGCCTTCAATGTAGGCCAGATTGTACGAATTTTTGCTTTCACTGAATCAG
GCAAGTTAACTGCCTCGGCAGTTAACGCAGCAACGCCTTTTGACGCCGTCATTTGAAGTTTAGAGCGCCAGTG
CTCAAATTCTTCGTCGACCTTGACACCTGAATCCACCCATTTAATTAAACCTCGCCCATGAGCATCGCCTAAAT
ATCCTTCGTGTACACTATCACGGCCTGAATCAAAGAAAATTGGGCGCAACTCTTCTGGTAGCTTGGTAAATTCC
TGAATTTTTCCATTATCATACATCATCATGCTTACTGTCATTTCAAACATAAAATCTTTTTCGCATACAGCATGC
AATCCGAGGGATTCAGGTTTCTTGGGGTTTTTAAAGCTCGTTTTCTCTCTGGCGCGTAAACATACAATGATATG
CATATTGCTCTGTAAAAGAGCGCTCATGAATTTTTTATGTTCAGACTTGGCTCGCTTCCAGTCCGCCATTGCCTT
ACCTTGCAATAGTGGTTGCTCTGCAATATCATTGCATCCTCCTTCACCTTCCCATTCGTGCGATCCTGAATCAAT
AACCAAAACCTTAACGCCTGCCTCCTGAAATTCCTCGATAGCCTGGCGGTAACGAGCTGGACTAAATGGCGCA
TACAAATCAGCGTGCATAAATGGACCGTCAAGCTCTCCAGAATAAAGCCTGCCACGACCATTTTCAGAGTCAA
GAAATCCAATTTCTTCTGGTTTATCGACCATACCTCGTGCCATCTTTAGTGCCGTAAGCGTCTTACCGCTACCA
GACTGACCTGAAATGCCAATCACTACGCGTGAACCAGAGCGCTCGGCTGGCTTAATGTTTAAAACACCCATCA
CTTCACCTCATTTACTATAAATTACATGTTAAATTGCTTTTTGAACCATTCCGGCGTTTCCATTTCGATGACCGG
ATTACCCATTGAGTAACCGGGCCATGAATTGGCTTTTTTGCATGCCTTATAGATTTCCATCGCGCTGCGCAGCT
GAATGCGACCAATGCGTAACTGCTCATCCGTCAGGCGAATCAGCGCAGGGATGAATGGGGATTTTTTCTCCTG
CACCAGAAGATTTACAGAGCGCGGCGCATGCCCGTAAGCCTCGACAAACATATCGTGTTGCATCGCCATCTTC
ATAAAGTACCCGAGCCGCGCAGCATGGCGGAAAAACTCATCAGGCTTGGCGCTCACCGCTGTTTTGTAGTCAA
TGATGTCTCCGCCGCGTGTAAGGCAGTCAAATCGCACCTTGGCAGGCTCGCCATTGAGTTCTCCAAGGATGGA
CACCTCAGTATATGCGCCAGCCAGCAAGCTGCTGTAATAGCTGTTTGCGTGGATTACGGCTCTCATCTGCATGA

TTGCGTCATAATCATCTCCTTCCAGCAAAATTCTGCCTTTCGCATTGAGTTCAGCAAGAAGGCGCTCTTCATCA
TAGATTCTCACTGGTTCTCCAGTTGAGCGGATGATTTTGATAACCTCAGCCTTTGTCTTGCCAGAAAGACCTTT
GATTCCGCGCTCTTTCGCCCATGAGTTCATGTCGCTCACGGTAACAAGGAGATTATCTCCAAAATCATCTTTCG
TAGGCATGCGAGCATATTCTGCATCGAAGCACTCAGGCTCAAGCAGAGCTGTATGGCTACCAGTACCGAAAAC
AAGCGCTTTCGATTGCTCGTCTTCTTCATCCTTGTAACGCCATGCTGCTGGGCATCTGTCATACAGATTCCACA
AGCCAGAGCCGTTGATGTGCTCTGTGTCAGCATGGTAATCCTGATTGCTTAATTCATTGTTAAAGTAAACCTTC
ATCCATCACCTCACTTGTTATTGTCAAATGAATCTACATCAAACCACGTCATTCATCAAGCCCAAAATAGAATA
ACGTCGCTTTTTTTAGCTCATCAAGACCATAAGCAATGGCGCCAAAGTGACCCTCTGAGATAGCCTCCTGCAG
AACCTCAATCTGCGATGGCGATACTTTTGATTTTGTCTTATCACGCCGCTTCAGCTCAATCAGGCCGCATTTAT
GATTAATGCCGTGCGTCAGTATCACGTTATCGCTTACGCCGCTTCTTACCCCCATTCTGCGGCGTTTTTCGACG
AATTGCGGTCCGCTCCTTGTTCCTGTCTCGTTTGGAACATGAAACCAAAGCACATCCGGGAAACGATGCTGCA
TCCATAATCCGTAGCTAATCTGGTCGGTTTCCTCTTTCGGGCAATCTCCACGATAGCCGCTGTCAAAAACCCAT
ATCCCGCTTTTAAGCTGCTTCACTCTGCTCTCCTGTGAAATCCTTCCTGTGGATGATATCCCGCCCCTTATCGTT
AAACCGGTGAGTGATGCGCTTCGGCGCGCGGATAAGACTGGCATAGGTCATGAAGGTTTTTGCATTCTGGCAA
TCCATCATCTTTTTCATCAGCGATTTATCCTCCAGATGCGGCAACAAACCCTTCATTTTAAACATATCACGCAG
GTGTTTAGGCTTGCCGCCGAAAGGATAAAATACCTCATTGGCCCAGCCGGTTTTGCCGTCAGCTTTCACGCATC
GGTAGCGATATAGAATGCCTTCGCCGTCTTTTGTCAGTTCAACTTTAAAATCCTGAACGTCAGTCCATTCATTA
TCGGTGTACGCCCGCTCATTCAGCGCCGAATTAGGGTCACGCAGCACATGGTCGCAACAGCGGCAATAGCGCG
CTGTAGGGTCGTTTTTAACTCCACAACCATCATCAAAAATGCGGATTCCGTGCTTATCAAAACCGCACCGGAT
AAAGCTAAAAAACTCCTCGCAACGACCATCTGCTGAATTGTCATCTCGTCCAATACAACGGCGCGCATAAGGG
CTGTTCATCGTCTGGCATTTAGGGCATGGAACCTGCTCACCACTTCGACTTGATCGCTGCGCTTCAGCCTCCTC
AAGAATAGGGTCTTCATAGAGGCTCCCCAGCTCAAACATGGTTCCAGTGAAGTCAAGGCAAAGATGATTTTCT
TTAATCAATCCTGCTGCAATCTGGTCAGGCTTAAGCAGGCGCATAGGGCGTCCAAGCAACTGAGTCAGCAACG
TAAGTGACATAATTTTTCGCAAAATTACGGACGTATCCCAGTATGGGATGTTAACGCCGGTGGTGAGGCAACC
GATTTGCAGAGTGTACTTCTTGCGCCCGGTAGCCGCATCTTTGAGCGCCTTGCGTCTGGCTTTCATGCCCATGT
CTTCGGTGACTATTGAATAGCTACCCTCTGGCAGGTATTTTGCTGCTTCTTTGCAGTGCTTTTTCCCGGCGCAGG
TAATCAGTACACCAAGTCGGTTTTTTGTCAGTTCCATAACATTCAACATGATTTTCTGTGTCAGCGTGCCCTGC
TCAAGGATTTCCTTCTCCATCTGCTTGAGTTGCGCATCGGTAAAGTCCTGCACACCATCCACGCCACTGCTTTC
AAATTCATGCAGGTCATATTGCAGATCGTCAATGTCAGGCATCCCGAAAATAGTAGGCACCAGATAACCTAGG
TCAACCAGATATTTGGTGCTGATGTTGACTATCTCATGCTTCCAGTACGGGCCTTTGATGGAATCAGTGCCACG
GAACGGGCTGCCGGTGTAGCCGATGACAATTACCTCATGTCCATACTTCTCTTTGCAGCGGCGGTTAAGCTCG
GTCATGATAACGCCGTACTGCGTTTCAGGATGATCGCTTACGATGTCCTGCCAGTTAACCTGATGGCATTCATC
AACCAGTATGTAGCGCGGGGAAAAGTCTGATAGCAGTGATTTCGTCACGGTGCCATCATCGGATGTCTTATCA
AAAAGACCATTTATTATTGTTCCCTCCGTTCCCGCTATAAGCGGATAGGCATATGCTTTACGCCCAAGTGAAGC
GCTAAACAGAGAGTTTTTTACGCTCAGATTCCAGAGTTCCTCTGCATCCTGCTCAATAATTTCGCCTTGTCGCG
CGATGACAAGACCCTCCCATCCCATATCTTGGAAACGACGGGCAATCATGGCTATCATGATGGTCTTACCGGC
GCCAACTGAAGCGGTAACATAACTTGGCTTTGGCTGCTTACCAAACTTGCGGATAACCTCCGCTGTCTTTTCAT
ATACCAGCCACTGATAGGGGCGCGGTTCAATTCCCCCGGTATGGATGCTGGCCCGCAGCTTATCCATATCCAG
CTCAGCAATCATTGCATCAATTTTTTGCATTTTGAATCACCCAATTATTTCAATCGCCCAAAATCTTTGTGGTAT
TTTTTAGATGCTATTGCATACGCCATTGCGGCTGCTTCTTTTGTGGCATGCCTTCCAAGCCATATTTTTTTATTA
TTAACCCATATCCAGCAAGTCCATCTACCTGTCTGCTTGCAAAAACCTACACCTTTCACTCCTGATGTGTTGTG
TTTTGGGGTTCTCATGTTTCTAACATTTTCGTTAAAGTTTGCCTCTCTAAGGTTTCCTATTTTGTTGTTTAATGGA
TTCCCATCGATATGGTCTATTATTAACTCAGGCCAATAACCATGCACTAACGCCCATATTATTCTATGAACCTT
TATATCAAAACCTAAAAAACCAACCTGAAGATATCCACTTACAGTCTTTGAGCCTGCAATAGCACCACGTTTC
ACCCTGTTTGTTGTTGTTCTTTTCCATATGAGATTTATTCCGTCATATGAAAAATACTCCCGCAGCGTGGATACT

GGAATTGACCTCTCTTTTGACTTCATCAATTCGAAATCATTCACTTTACGTTACCCTCATTCAGTGTTACGCTTA
AAGCATACAAACAGTAACAGATGGAGTCAACATGTTTTATTGGGAAGATATACGACAAAGGATGATTGGCAA
CTGGGAGGCCGCGCTGCTATCAATAGTGAATATTGACAGGAAGGTTTTTAATGGAAAGCATCAGCCTTGCCCT
CATTGCATGGGTAAGGATAGGTTCAGGTGGGACAACAACTTCGAAACAAAAGGCGACGGTGGCGCCATCTGC
AACCAGTGCGGAAACGGCAGCGGAATCACCTGGCTAATGAAGCTGTCAGGAATGACGTTTCCAGAGTCAATG
GAGGCACTGGCCGGATTTCTTAACATGCACCCACGCGAAAAACTTGAGGCGATTAGAAAGCAACTTCCGAAG
GTCAACCATGCTTATGACTACCTGACAGAGGCAGAAGTGGCGGCCATCATGGAAAAAGCCGGGGGCGACACA
ATAACCGGCAAAACTGGTGAACTGGTGGCGATACCGCTCTATATGGCTGGAACCATGTCGCCGTGCAACGTGG
CTTTTCTGGCAGACGATGAAAGCGTATCATTTCGCGCAGGTTTCAGCCAGGAGTACACTCGCGGAAGACTTAC
GCGAGGTGCAGTGACACCCATCGGCGATAAGACTGAATGGACATACCTGGTTGCGGATTACTTCGATGCCTGG
CGAGCGCATCGGCTTACCGGCGCTCATGTCTGGTGCTGCTGGTCGCCGGAAAATATGTGGGAGGTTGTGCGCA
ATGTGAGCGATGAGCAGCGGGCTAAACTACGCTGTATCATTAATAATAATTTCGATGAGGTATGCGCCGCCGA
GAATACAGGCCTTCCAATACTGATTACTGATGACGGGCAGGATATTCGTTACAGTGGCGCAATCAGAAAAAG
GCTGTATAAACCAGAAGAACTATTTGAAGCACTAAAAAACAAACCCTCCTGATGGAGGGTTTTTGTTCAATAC
AAATCTTCGCTAAAAAGTTCATCACTAGGCCATGTTTTTATTCTTACTACATTTAACTGACCTGCATCCCTTGCA
ACCTGCAATGCCTCTTTCCTGTCCATAAACACACCGAATTGGTCAACAAACCCCTGCACTTCAGATTCACGCTT
TATGAAATCCTTATGTGGCTCCATATTCTGACGCATTACCGGGCAGAAATGTCTAACACCTATAAAAACCATA
TCAATCCCACCGTATCCGCAATCATATTTATTTGCTGCGCATACAACTCTTCTTTGTAATCTATTCATCATTTAA
GCCTCGGATTCAGGTAAACAGTATTATTGAGGAATACGCAATATCCATCCTCTTCAAGAGATGGCAAAACGTT
TGACTTGAGTCTGTCATAAATATGCGGTATACCTTTGAATGGTCTAACGTTTTTTAGTGAATCATACAGCCATT
TCACTGTGACATTTGTCTTTCCTTTTTGCGCAGCTGTGCGCAATTTCTCAGCAACAACGTCAATTTCTGATTTCT
CGCCAGCGTATCCATTTGACTCAACTGCATCGGTGAATGTTTTTGTCAATGCATCATAAACGCTTATGGCGCGG
CTTATATGCTCCTCACCAATAATTTTTGAGCGACGACCGCCATCGCACCAGTTTTCAGCGGCATGGAATATTGC
CGAAAGTCTGATTATCTGTTTGTCAGCCTTACCCATGGCGCCACGAAGCAAAACGTGATCCCATTTTCCACCTG
GCAGGAAATTCTTTTCCCACTGGTTTCGTAAAAGTCCAATCATACGCTGCGATTCTTTAGCGAGGGAGAAAAC
AACCTTCTCAGAAGCCACAAGGTTATGCACAAATCTGGCATATTCAGCTTTTAATTCTTGTGGCATTGGTTTGC
TTACCGGACAATCATTTTCAACATCCCAATGCTCACGATAACCCAACATTGACTGCTCGCGAAGCATCAGGAA
TCGCTCTGACAACCCGTTACCCCTGTCACCAGCCGACAGAATGGCATCAATACTTTCATCCTGTGCAATGACGC
TGATGTTACCAAGAACATAGCCAGATGAAACACCACGACCAACGCGAGCTGAACCAACAAACCCACCGTCCC
AGCCTTTCAGGATGACCTCTGCGTTAGACTTTCCACCATCTTTGCCATATGAAAGGCCAAGGCAGGTATTTAAA
ACGCTTGCTTCATCACTTATCAGGTTGAAAAATCCACCTTCATGAATTGCCTGGTGCTGCACCGCTTCAGGCGT
TGCATCGGTTAACGGATATGTGATGGTATAAAGACTTTCCAGCTTTTCCTTTTCTTTCGCGATATCATCTCCAAT
GATGGCTTTCGCGTTCTGATTCGTCGCCTCCTTGTAAGCCTTCATCAGGTCTTCAATGCGAATGTTTATTTTTAC
AATTTCCTTCTCCATCTTTTTTGACAGGTTGTCATACTCAATTTTTATCGGGTTCATGTGCATTGAGTTTATGGC
TGTCTTTCCCGCTGATGGAGGCTGTGACGTCACCACATACAAGGAAACTGGTAACTCAGAGCCATAGTATTCA
ACGCTAAAGTTTCTGGTCATGGCACTTGCTACGCAGCCAAGCAGATGCATGAATGACGTGCTAACGGGGAATT
GCACAGCCCTCGCTGCCGCAACTGAGTAGCGCGTTATCAGGTCTTTCCTGTTGTCACTGGTAAGCTCTATCTCT
GAGTATGTTACATCCTTCTCCTGACCCTCCTTGATTTCCGGCCACATGTTATGGCTTGGCGTCATGCCATGGTG
AATGGCTACGCGCGCCGCAGAGGTGTGCGCTTCTCTTGCCTGATTGAATATTTCCTGGACTGCAATCATTTAAA
CCTCGAATTCAGTCGCAAATAATTCGTGAGGGTTTTCTGACAGCCATTTCTTTGCCTTCAGAACAGCTAGGTTC
GCTTCTTCTGCGGTGTCGAACATGCCAATGTCATATCTCTTTTTGAGCACCATAACGCGCGCTCGAAATTTCTT
CGACGCCTTGTGATAGTCAACGCCGTAGTAACCAGTTGTAGACCTTGGCGATGCGACCCATCTGGCACCGATG
CGATTATCATTTCTTTTCCGTTTCAATAAAACGCCGTGATTGCTCATCCAACTTCCCTCCACTTTATTTCCCTAA
AAGATACCAGAAGATTCCCCAGCACGCAATGATGCTTTCATTTAGGCGGATAGGGTTAACTGGGGCAGGCGTG
GGGCATGCCACCTTCCCCACGCGCTATCTATCTAAGTGTATGTAATATAATAATAATAGTAGTAGTTGGGTTAA

TGGGTATTATTATTACTCATATTATAGAGTATAATTTAACATTTAATTAACATATGTATTATTGGATGTATTTAT
AGGGGGATACGGGTCAAAGTTAACCAAGTTAACCCAAAAAAAATAAATCAAATAAAAACATGCAATTAATAC
AAAAAATTTGGGGCAACTGACTTGCCCCACGACCTGCCCCAGGGGCAAAAAACTACCCCACGTAGACATTTTT
TCATCACCTTATTGACGTAGATTGCCATGCATTGTATAGTTACCTCACCAACAATAACGAGGTGATGAAGATG
GAACAAAAACCAGCAGGCCGTGTGGTATACAAGCGCACTCATCTGGAGCCTGAATTTGGCACGGTGAAGGCG
CAAAACTTTATGATGGCACAGGCAATGCATGCATACAGCAACGGCAAGCGCGTCTGTCGCGTTTTTAGTGGCG
AAGGCAAGCAGAGAGTGCTTGAGCAGGTTATCGTGTCATCTGGTGGAAACTAAACCGGTTTAGCAACGATGA
ATAAATTACTGGCAGTGGTTCTACTGGTTATCGCTAACTCAGCAAGCGCGGAAACGATATGGGTAACGAAGTA
CGCGCTGACCCGTGGCATTCAGAAGTACGAAAGCGCACAGCTGTTTGCCGATGGTCAGGTGGCTGTGGTTGGC
GATGTTTACTTTAAACGTGGTGAATACTGGCTTGATGAGCAACAGGCAAAAGAGCATGCAGAGACTTTGCGGC
AGCGCCGTGTATCTGCATTGATGCGTGAGCTTGAGCGCTTACAGGCGGTTAAGTGAGGATTTATGGATATCGA
AATTAACGAAGTTCAGGAAATTATTAAAAGCCTTGAGAGTGGTGGTGAACTCTCAATCAAAGAGGAAAAATA
CCTCAATGTTGCAAAGATGTGCGTGCAGCTTGCTGCGGAGAATGTGGCTATGCGCAAAACACTTGGTGAGGTG
GTCTATTCATCCGAAGAGGTAGAGCATGACGGATGCCTTACATTCGTAGTTAATCCTGACTGCGTGCATGATG
CGGCTGATTTACTGGATGGAGAAACCCCCACCACCGATCGCATCGTAGCCGGGATTAAGGCTGATGGTCGCGC
AGAGGGTATCAACTTTGCAGCAAGCCGCCTTGCTGCTGCTTTCAATCACGGATTCGTTGATAAGCCAATGGCA
GAGGTTGGTGACGTGGTACGCATGATACTCACCGCAAAAGAGGATTTGGCGAAAGACCCGGTCTTAGCCGCC
GATGGACTGTCAGGAGAGTACGCAGAAAAATCCCTGGCTGAATGGGAAGCTGCGCTGAGCGATGGGGCCAGC
TATGACTGATATCACCGAACTGGCGCAGAGCCTGAAAGCGGCAGCCAATACAACCTCCGATGCTATCGACCGC
CTAAAAGCATTCCCAGGCGACGAACTAATTGACCTTTCACAGCATGAGGGTGAACAGGTAGACATTGATATCA
CAATGATCAATGAGTGGTACGAGCTATCAAGCCCGGCAAACATCCTCGCGCTGCTAGAGGCGCTGGAGAAGG
CGCAGTCCAAAATCATCGAATTGCAACAGGGCTACGAAATCGATCCGCGCAATCACGAAATTATCGACCTGCA
TGAGCGCATCGCCGAGCTGGAGTCCCGCACCGTGAAGTTGCCAGAGCTATGCGTGGGGGTTGTTCAAGGTGGA
CATGCGGTAATGGTGCCATATCCAGCAGGACACTGGTTTAACAAGACAGCGATTTTGGAGATGTTGGCCGCCG
CTGGCATCAAGGTGGAGATTGAGTGATGTGGGTGCTCATTATCTGGATGTTCGGCGGTTACGAAAACCCGACC
ATTACCACTCAAGAGTTTCAAACAGAATCCGCCTGTCGAGCTGCATTTGCCGAAGTGAAAAAGGTAAACAATG
CCGACGTTTCTCTACGTGGCGTATGCACGCCTAAGGGTGACCAATGACCAAATCAACCATAACCAGAGAGCGC
GCACAGCAAATTTTCCTGGGCAACGGACCAGAGCCGAGCGCATCAGAAGAACGAGAGCTGGCCCGCATGGCG
CTGGCCGCAATGGACAGTGAGCCGGTGTCACAAAATGAGCCCATTAGTTTTGACGAGTGGTCACGAAAATGC
GCTTTGCAAATCACGCTTTGCGACCCTGATTTTCGTGAAAAGGCTAAGTACATCTGGGATTCAGCGCGCGAAA
CAATGCATCCAGCGCCGGCATCAATGAAAGACCATCAGATTCGTGAGTTGGTAAACGAGCTGCGCGATATCGC
TATCGAGCACCACAGCACGCAGCAGTTACGTGAACGCATTGCCAGAACTGTACGCGCCGCCATGCTCACAGCC
GTCCAGCAGTCACCCGGCAGAGAGCCCGCTACCGTGCCGGGTACATGGATTCCGGTAAGCGAGCAGATGCCG
GAAATTGGCGATATCGTGCTTACCGCAATGGGAGGTGTGGTTAACGTTGGCGAAATGGAGTGCTCTGCTGCAA
ATTATCGTTTCTTCACGTCAGTTATTTCCGGCCGCGAGTTACCGGCGACTCACTGGATGCCTCTGCCGGCCGCT
CCGCAGGAGGTGAAGTGATGGAATGGTATTTCTCCATCGGTCTTGTTTTGTGGATTTTGGCGCAAATAACAAG
AGCAGATGAAACAACTGGCATTAAGAATAACATTCTGTGCTTGCTGCTCTTCATCGTGTTGTGGCCAAAGATC
ATTATGACCATTGCTTGCAAATTTATCGAGGAAAATGGGTTGATGTCTAAGTCATCAGCAGCAGTAAAATCCA
TGAAACCCTACATCATCCGCAGGATTATTGCCGTAGCCTGCATTGCATTCTGGATTGCTGTTGCACTGGCCGTC
TACTTTATCTTGAGGTGAATCATGTTGTGGAGCGACATTCAAGCTGCATGCGAAGAGGCTGATTTTCTGTATGA
GGAGACCGGAAAGCATCATGCTGTTATTCAGGTAGGCAGCATGATGATGGTGGTTGAGCATAACAGCATGCTT
CGTCATATGTACTCAACGACGAGGTATCAGTGATGCCGCAAAAATCAAAACAGGAGGTGTGGCAGGCCGCGC
AGATTGAAGGTGTTGGCCACTTCATAGCAGCAATCGCCAAAGCCTTTCCTGATGCGATCGAGGTGGTTCACGT
TCAAAGCAATAACTGTAATGTTTGGTGTTATGCGAAAACTGATGTACAATCATCTCATCAATCATCACCCACC
ACCCTTTAACCCGCTTCGGCGGGTTCTTTTTTTGCCTTATGCACCATGGTGTAGAATCTGATACAATATCGAAT

AACGGAAAAATAGCGGAATATTGATAGGTTTTATCTATGGCTAAAAAACTATTCAGTAGCGAAAATCAACCAC
AAAATAAGCGCGGGAAAGACAAGCGAAAGCTGCTTGTAGAGGCTCTTGAGCGCAAGGGATTCAGCGAGGAA
AAGCTGTACGATACCATTATTGAAATGGCAATGATTGAGCGCGATACCGCGATGATGAAAGAGCTTATCGTGC
GATTTAGCCCCCTACCAAAGCCTGTGGCCCCTGTATTTGAGGTTGATTTCCCGGATGATGGAACTCCGGTAGA
GAAGATTGATGCAGTCATTCGCGGCATTGCATCCGGCATTATTCCCGCCGATATCGGTAAGATGTTTGCCGAA
GTTATTAAGACCGGCCTTGATGTCGCGGAAGTAACAGAGCTTGCGGCGCGCCTTGAGCGACTGGAGAAGTTAC
TGGAGCAGCAACAGTAACTGATTTATAATAATCAATACGGCTAGGGGGCACCCGAAAAGCGACTCATCACCG
CCTGCCGCATCCCACCAAAGATGACCCTGATGAGGAACCAAAATGATCACTCACGAAGAACTGATTCAAGTA
ATGACGTATGACCCACAAACTGGTGTTTTTTCATGGAATAAAACAAGGCATGGAATAAGAAACATGGATAAT
GCGGGGTGCGTAACCAAGCGTGGCTATGTGAATATATACATTAACTACAAAAAATACCTTGCGCATAGATTAG
CGTGGTTTTACGTTTACGGGGAATGGCCTTCGAAGCAAATTGATCACATTAACGGCGATAAGTCTGATAACAG
AATATCGAACTTAAGACTGGCTAATGGCTCTGAAAATCAACTAAACAGATGGATAGACTCAAGGAATACATCT
GGTATTAAAGGTGTTTGCTGGCATAAAAATAACAAGAAATGGCAAGCTAGCGCATCACTTGGTGGTAAGGAG
TATTATCTTGGCCAATATGTAAACATAAAAGATGCGGAGAAGGCTGTGGTTGATTTTAGGGAGAAACATCACG
GGGAGTTTTGTAACCATGGCTAGGAAAAGGCTATCAAGTATTGCAATAGAAAAGCTCGAGCAGGCGGTTGGA
AACTCAACTGCAAAGCCTGAGTCCGCTGTGTTTGGCATTGTTGATAAACTTCTTGCCGATGGCACGCCAAATGT
TATCAAGCGACTTAAAATGACTCAGGATAGTGTATCAGAGACAACTGAAGAGCCAACAATATTAATCCCTGA
ACGCATGGAGCTTTTGCTTTATCCGCGCCGGTTTAAGGTCTTCTATGGCGGAAGGGGCAGCGGCAAAACGGCT
AACGTCGTTTCCTATCTTATCGAAAAGGCAAGATTCAGAAATTGCAGGGTTGGCTGCTTCCGGGAAATTCAGA
ACTCAATCAAGCAATCAAGCTACACAGAGCTTGTTGATGAAATTAACAGAAAGGGGCATGCGCATGAATACA
GGTGCGTAGATGGCGAGATAACACACTACGGCACGCGGTCAAGGTTCGTGTTCCGGGGCATGTGGCGCAACA
TCACGGCCATCAAAGGGATGGCTGGGCTTACCGATGTGTTCTGCGAGGAGTCGGAGAACATTAGTCAGGTGTC
ATGGGATACACTCATCCCTACAGTTCGCGCCTCTGGCTCTGAAATTATTATTGTTTTTAACCCCAATCGGGAAA
CCGATCCCACGTGGACAAACTTTGTTGAGCCGTATGTGGATAAGATGGTTGATGGGATATATCAGGATGATGA
TATTGTCGTTGTGAACGTTAACTACATCCATAACCCCTGGTTTACCGAGGAACTTAAACAGCACATGAACCAG
ATGAAGGCGGTTGATTATGACCGCTATCTGTGGGTTTATGAGGGGTTATTCAACCGGCGTTCAGATGAAGCTG
TGCTTGGGGGTAAATGGCAGACTCTTGATTTTGAGCCGTCGCCAGATTGGGGCGGGCCTTACTATGGTATTGA
CTTTGGTTTCTCGCAGGATGCTACGGCAGCAACTGAATCTTATGTCGAGGATTTGGGTGGCGGAAGAAGAAAC
CTGTATATCTACCGCGACTTTGCAAAGGTTGGTCTTGAGATAACTGATACGCCAGAAGCTATGCGACTGGCAT
TCCCGGGTTCAGAAAAATACAGATGGTATGGTGACTGCGCAAGGCCAGAAACAATAAGCCATATAAAGCGCT
CAGGATTTGATATCCATCCGTGCGCAAAGTGGCCTGGCAGCATTGAGGACGGAATCACATGGCTCAGGGGTTG
TGACAGGATTTTTGTTCATGAAAGATGTAAGACTGTCATTGAGGAGTTTACTCTTTACAGCTACAAAGTGGAC
AAGCTCACGGGAAATATACTTCCTGATATCATCGACAAGCACAACCACGCCACAGATAGTTTGCGCTATGGCC
TTGGCGATCACATTGTACAGCGCGGCTCTGGGTGGATTAGGCGGAGCAGGAGATAAGAAAAAGCCCCGATAA
AGGGGCGATCTATTATCTCAACTTGAACAATTTTTCTACTGACGGCTTTGAATGGCCTGACCTGCCATTGGCGC
TTAGGCTGCTTTTTACATCAAACTCTGCAATGCATTCAAAGTCACCTGGAGCTTTATATTCACTGATATAAACA
TCGCATCTTCTTGATAAATTCCTAGTCCAATCCCAAAAATCATCATTATTAAACGAATCCTTGTAACCAGTGGT
GCCAGCATACGGAGGGTCGCAGTACACAACTGAGCCACTCTCTATTTGCATCTCATCATAACTAAGACTAGAG
AAAATGACGCCTTTTAGGTTTTTAATTTGCTGGGCTGCATTCTTTATTGCTTCGGCCTGGTAATCCCTCATGCCA
CATCTTGTTTCTACTATTCCAGCATACCCACCGAACCATTTCCCGCTGTACGAGCAAGCTATTCCAGCCCATCC
TATAAGGTGGTCATCACCCTCGAGATTCTTGAGATTCGCATATTCATCTCTGCTTATAAACTTAGGATTCCATC
CGGCGCATAGCGCCTTAAATGCAGCAATAACGTACTTATTACTGTCGTTAGCAAATCTTGCTCTTGCATCTCTG
ACGCCATCAATCATATTCATGCCGCCAGCAAATGGCTCATAATAGTTCCTACCACCAGTATTAAGTAAAGGGA
TGATTTTATTTTTAAGTCTTGCTTTGCTGCCCATGTATTTCATAGATAAGCCTCATCAGTAGAAAACAATAATCT
ACATCAACCACGAATCAACGTCAACATTTATAATAAATTATTTGTAAAGCCAGCAATGGACCAGGCATTTCCG

ATGGCGCGTAAGGCTCGCTGGCATGCTGACTCATCAAGGCCGGAAACCATCAGTCACATCAAGCGCGCTGGCT
TTGACATTCACCCGTGCAACAAATGGCCCGGCAGTGTTGAGGATGGCGTTACATGGCTCAGGGGTTGCGACAA
CATCATCATTCACGACCGATGCGCAGAAATGAAAAACGAGGCTGCGATGTATAGCCACAAGGTCGACAAGAA
CACCGGCCTGGTGCTGACTGAAATCGTTGATAAATACAACCACTATTGGGATGCTGTGCGCTATGCGTTAAAT
GACTATATCGTACAGCGCGGCTCTGGGTGGATTAGGCGGAGCAGGAGATAAGAAAAAGCCCTCATTAGAGGG
CTTATTTTTATAATGTGCTCTGAGACCTTTTTCCTAGTTCGATGACGCAAGCCATTGCAGATTCTATGGTGTTGA
ACTCATACAGATGCTTTTTATCTTCCATGACATAGAACATTAATCGTCCAAATTTATTTTTCTTTGCGCTAAATT
GAATATCTTTCATCTTTGCTATCTCCGCTAATCAACTTGATGAATCAAATCTACATCAGCAAACAATCCACGTC
AACATTTATAATAAATTATTTGTAAAGCCAGCAATGAATCCCGGCATTGCGCATGGCTGCGTAAATCACGTCG
TCAGACACGACTGCCATTGCCACTCTGTCTGCGTCTATCTGCTGGTGCGATGCCATGATATCATCATAGAAAAT
ATCGTTAGCATGCAGCCACTCATACGCATGCTTTGCTCTCATGATAAGCACATCGTGACCGGCAGAGTAAAGC
GACTTAGCCAGCGCTACGTTGCCAGCAATATCATTTCCTTCAGCATCACGCAGCACGCCATCAAGCTCGAAAA
TGACACATTTCATAAGATTTCTCCGAGAGGTTTCATTTTAATCTACGCCATGCTAGAATCTACGTCAAGGCGCA
TTGACATAACTACATCACCGGGGCATCATGAAGGCATACTCATCTTTTTCGTGGGAGCAGAAGGAAAAAATAT
ACTCACTCGCAAGAGCTGGTGTGTCTGATGAGGCGCTGTGCGAAAGGTACGATGTGGATGAGGCTATTCTGCT
GCGCATGTATGATGAAGTGCTGTGTGAGTTGCAGCGGCGCCGTGGTTATAGTGGCCTGAAGACGATTAATGAT
TTCTTTCGGAATGTTGATTTAAATAATGGTGAGGATAGTGAGGATGGTGATTTGTGAGAGATATTGTATTTATG
GTTATGGTGAAGTTACCAACAATATTCGCCATCGGTGCGGCTGCTTGGCTGGCATTCAATAACAAGGAGGGGT
GGGGTTGGTTTCTTTTTGTTGCGGTGCTGATAACTTCATCCGTTAAAATTGATGATTGAGGTGGTGATGATAAA
GAAAAAGCTGCAAATGAAGATGCGCAAAGCCGCAATGGAGAAGTACACAAGGAAGATTGATATCAGTTTTAT
TGATGGCGTTGGCATGTACAATCACGCATGCCATCTTAATGCGGTAAATCGCGCAAGAGCAGGAAACAGTGCC
GCCGTGGTTGAGGTTGTTATGATTAATGATGACTCGGTTACAGCGCATTACATCAACATGCAGGAGGATGGGA
AATTTGTTGATTACACGCTTGGCTGGCACTGGTCGGGCGCTGATTACAGGTTTGTGCGATACGTTCCATTCACT
GAGTGGGGAGATATAACAAGTTCGCTTGAGCTCCTCAAGGCAGAACTTTGTAAGCCAGTCAGCAAATGGATG
CGCGCCTTTATGTTGACTAATAACGAAATGTGTTGAGGTGATTTATGATTATTGAAGATGAAACCAAAACGCC
AATGTGCGAAATAGCTATTCGGTTTGACTCTGAATCAGTGATTACGCAGGGGCATGACACTATTATTGTCGAC
AAACGCCAGGCTGCGCAGCTTATCGAAGTCCTGCAACGCTGGGTTGATGGCGGGGAGATTGAGTGATGAGCG
TTTATTTTTACCGTGGTCACGCAGAGCGACACGGAAGCACTGAGAGCGCTGGTAGTTTTTGCGGCGTCGTTGT
GGCAACTGATGCTCTTGCCGGGTATGAGTCGGCAATGAGAGAGCAGATTAAGTCAATTAGATTGATAACCGAC
TCCTGCGCAGCTAATTTCTCTGTTATTTTTGACAAATTCGAAAAGGTAGAGTGATGAAGCGATTCCTTTTTTCA
TGGTTATACCTCTTTGTGATGTACATGATATTCCTTGCTCTGTCCTTCCCGGTCTGCTGGCTCATCAAATGGCAG
CCGCCGATGCTGAGCGATATCATGAACATCAGCGTGCTGCGCATTACTGTATTTCTGCTGGTTGCGACTATGGT
TGGCGCATTATTTCCTAGTAGAGTTGATTGAGGTGAAGTGATGTCGTATTTCTTCCTGATATTCGTAATCACAA
GCAACACGTCCAATATGCAGGTGGTGCCAATGCAAAGCATGGAGCAGTGCAGTGCGGCCATTACGGCGATGA
AAATCGCTGAGGGAAGACGGACGTGGCGTGATGTAAGTACAAATATTAACAATTTGCAATGTGTTGAGGTGA
AGTGATGACAAGAAAAGAGATTCATGATGTGGCGGTTGGTTGCAGGAAGTGGATTGAGGAGATATGGTTTAA
GCCTGGATTCAGTGGAAAGCGCTATAAATTGCACGCCAAATGTCGCGGTTTGGTTCTTGGGGATAGAATAGAG
CTTGCTATATTCCATAACAATGACCAGGCCCTCCAAGAGAGAGCCTTTGGTGACTGATACCATGCTATAATCC
CATCCATGCGATGGGATTTTTTATGGTGACGAAATGTCAAAGTTAGAGGCGGTAAACGCCTATATTCAGCAGC
GAGTGGCGAACAATAACAGGCTCATAGAGCGGCAGCGTCGGGAGCTTGGCGGGAAGAATATCGACCACAAAC
ACGACAGGCTGTGGCTCGAATGCGGCTACCCTGAAGAAATCACCGCCGAGATGTTCCGCCATGCCTATGAGCG
CTATGCACCGGCAGCAGCTGGCGTCAATCGCGTACTCGATAAGTGCTGGCAGACTCCGCCGCAAATCCTCCAG
GAAGGCGCCGATGATAAGGCCAGCACTCCGTGGGAGAAAGCAGCCAATAAGTTGTTTAAGCGTGCTGCGCCA
TTCATCAAGGATGCAGACCGCCGCAACCTCATCAATCGCTACTCCGGCCTCATCCTGCAAATCCGTGACGGAA
AGCAGTGGAATGAGCCGGTAGACACCACGAAAACAAAACGCATCAAGGATGCTGCCATTGTCCGTTATATTC

CGGCGTGGGAAGAGCAGCTCCGCGTCAGTGAATGGGAAAATGACGAAGCCAGCGAGGACTATGGCCAGCCG
AAGATGTACGAATATCAGGAGTCGGTAGTCGGCGCCTGCAACAGCGACGGAAAACCAACGCGTTCCCTGAGC
ATTCACCCTGACCGCATTATCGTATTTGCCGAGGGCGCGATGGATGGCTCTATTTATTCCGGCGTTCCGCTCCT
GCGTGCCGGGTATAACCACCTCATCGACATGGCGAAAGTCACCGGCTCAAGCGCAGAAGGCTTCCTGAAAAA
TGCCAGCCGACAGCTCAACGTTAATTATAATAAAGACAACGTTTCCGCTCAGTCTCTGGCGCAGCAAATGCGC
GTGCCGCTGGAAGAGTTGGCGGATGTCCTCAATGAGGATGTGGCGCGCCTGAATGAGGCGATTGACGCGGCG
ATGTTCACGATGGGTGCTGATGTCAAAGTGCTCTCAGTGACGCCAGCAGACCCGAGTCCAACGTGGACTATTG
CAGCCAACCAGTTTGCGGCATCCATCAAGAAGCCATTCACCATCCTGTTTGGTCAGCAGACTGGCCGCCTTGC
ATCCGATGAGGATAAAACCGACGACGCCATGAGCGCCAAACAGCGCCGCGAGGACTGGCTGGATTACATCAT
CTCGGTATTCATTGACCGGATGATTTCCTTCGGCATTCTCGATAAAGCGCCAGAGAGCGGTTATTACTGCAAAT
GGGATGACCTGCTTGCACCTTCCGAGCTGAATAAGGCAGACCTGCTGGTCAAACTTGCCGCTGCAAACAAATC
CGTATTCGACGCAGGCCAGATGGCCCTGATGACCGCTGATGAGATGCGCGGCATTGTCGGCATGGAGCCTCTG
GAAGAGCAGCTTCCTGACGGATTGCAGGAGGGTCAGCAGCAAGACCAGCAACCGCAGCAAGACCAACA
GCAGGACCAGACCGATGCACCTCCTCAAAATTAATGCCCGGCTTCCGCAGCCAAAATTAAGCATGAGCCTGAC
AGACCCACTCGGCGCAGTGGGTCGCGTCAATAAGATGGTGCGCGATGTTGACGCCAGATATGTGACGCTAAA
ATCTCAGGTTGCCGGGTTGTTCAGCACCATTCCAGTGGCGACCGGCAATGCTGAGGCTGGAAATTATTATTAT
GATTTCTCCGCCTACCGCGCATCGACATTCTTTGATGAACTTCAGCGCATTCTTGATGATCAGCTGCTGGAAGG
CGATGACTTCACGCATGGCAGGATGTGGGCTTCATCCTACGTCAGTGACGCAATGTACGCCGGTACGCAGAAG
GCAAACTTAGACCTGAGCGACCTGTCGTCGGCATACAAAGACAGCAGGCCGCTTGCTGAAATACTGTACTCTC
AGCCGTATCTAGACAGGCTTCAGCTTGCGTACACGCGCACGTATAACGATTGGGGTGGCCTTTCAGATTACAC
GCGGCAACAGGTGGCGGAAGTCATCACTGCTGGCATTGCAAACGGCGATGCGCCAGGAGTCGTTGAGCAGAA
AATTGCCGACCGCATGGATGTGTCGCGCAGCTATGCTCGCTCGATAGCACAAACAGAAATTACCAACACCCTG
CGTGAAGCCAATCGCCGGGAAGTGAAAGAGGCACAGGTGACACTCGGCATGGATACCATCATGCTGTGGCAG
TCGGCACTCATGAAGACTACCCGCGTTAATCACGCCGCACGTCACGGTAAATACTACACACCAGAAGAGATTG
ATGAATTCTACAGCGAAGGTGCTAACCGTCGCAATTGCCATTGCAGCCAGACCCCTGCGCTTGTGATGGATGG
CAAGCCGGTGATACTCGAAAGGACGCAGGAGAAACTGGATAAACAGAGAGAAGCGTGGCAGGGAGCACATA
AAAAAGCCGCCTGATGGCGGCTTGTTGGTTATTTTTCTGCACCAAGTATCGCTGATGCTGCAAACCATACATTT
GCGATAATAAAATCCACATCACTCTTTGTTGCCACTGAGAATGCGAAGTAAAGAACACCAATAATGAGCGCA
ACCAGGCATTTCATTATCAATCCTCCAGCTTCACGCCGCGAACTTTACCGGCTGCGATGGCGTCGTAAATTTTC
TTGGCATGATAAAATTCGTTTGGTGTGTCAGCAATAAACTCAGGCAGCCAGTAATCAATATCCTCAACAGCAG
CATCGCGCCTCTTATCGGCTTCTGAGCTGGTGGGGCGGAATTGATAGTTTTCCCATTCCAGTGCCACCGCATCT
TTACCCGCCCAACCAAATGCCACACCACAATCAATGGCCACGCAGCGGAAATTCATCCACTCAGCAGAATCTC
CGTGCCGGAATTTGGCTTCGCACTCACACCCAACCTGAGGCAGCCCTTCTCCACCCCACGCCAGCGCAACATC
CTGCCCGATGCACTCATTCAGGTCTGATTCAGCATCAGATTCTGTGATACCAGCATAAGCATCCCATGCGGCA
GCGCGTACCTGTTCTGATTTCGTCGGCTTATGCAGGCTGTAGGCGATGATGTCTCCCCCTGAGTTATCACTTCT
CCAGAATGGATATGATGCATAACCACCTGCGAAATGCGCTTTCTGCGCATATTCCGTGCGACCATTCCTTAGCT
TGACATCAACAAGAACTCCATTTTCAATCGGGCATTCACCGCCACCCCACTCAATCCAGCCTTCATTTTTGGCA
GCCAGCGCGGATTCGTACTGTTCGCGGGTCACCTCTTCATCGCTGTCCTCGACCTCAATATCCGTGTCGATTGT
CCATTTTGGCGGGCAGCCATTATTCTTATCCCAGAAATATAGCGTACCCTCACCAGAGAGAAACCCGGCGCCA
ATGGCACCATCTGGCCACCCGCCACGCTTCGGAAGTTCTTCAACCAAAATATCAATCAGCTTCATTTCTTATCT
CCGTTATAAATGCTTTTCAGTTCACCCATTACATTCAGCCATGCTGCATGCTCATCCATTCCGCGCATTACCAG
CTTAACGTAGCGGTTGCGGGCCTTAAACATCAGGCGCGGGCACATTACAAGTCACCCATGCTTGATACGCAAA
GTGCAATTGTTATTGCCTTGATTTCTTTTGGTGTCTTGTTCGGCATTGAGTAGGCCATCTTCACTGTTGCACCAA
GCACCTTGTTGGCATCCACGCCGTACTCAGGGCTTTGCGCTACTGCCATTGCTATGCTCTGAGGTACGCCAGGA
TCACGAGCATCTGCCGCCGCGAATCCAACCTCACCAACCTTGTCGCATACCTCACCAGCCGACGCACCAAAGG

AAGACATTGCGATAATTGCTACTGCGATTAATTTTTTCATTTCTACTCTCCTGTGTTTGTGTAACTACATCATCG
CCCATGATTCAATCTACGTCAATATGATTATGATAAAATAATCTGCATTAAAGTGGAGGCACTATGAAACTAT
CAACGCGCGGAAAGAATTTAATTAAATCTTATGAGGGACTGGTTCTCGCGGTCTACCCTGACCCGGCTACCGG
CGGCGCTCCGTACACCGCAGGATATGGCCACACCGGGAGCGATGTTAAGCCGGGAATGAAGGTAACTCAGGC
AATGGCTGACGCATGGTTTGATAAAGACGTAGCGAAATTTGAAAGCGGCGTCTCGTCACTCGTCACCGCCCCG
ACAACTCAGGGCCAGTTTGATGCAATGGTGTCGCTGGCCTATAACATCGGGCTTGGTAACTTTGGCAAATCAA
CTCTGCTGAAAAAGCATAACGCCCGCTGCTACACCTGCGCCGCTGACCAGTTCCGGGTGTGGAATCGTGCTAA
TGGCAAGGTCATGAACGGACTCACCAAGCGCCGCGCAGCTGAGCGTGAGGTATACATGTCATGAAGCGCCTC
AGTAACTGGCTTATCGGCATTTGGGCGTCATTCTGTTCGCTGATTCAGCTCTGGCCTGACGCTATGGTTCATGT
ATGGGCATTCATGCCGGAAGACTTAAAGTCTGCCATTCCGCCGATTGCAGTCAAGGCCATCAGCTACAGCATT
CTCGTTGCCTCGCTGCTTGGGAAAATGCATGGCATGAAGAAAGAGATTAAGGCGCTGAAAAATGATTCTGCAA
ATCCTTAAAGCTAACTGGAAGGTTATAGCGGCTATTATCGGCGCCGCACTTCTGGCGCTGATTATCTACGGAA
AGTGGGTCAATTACGGGAAGGCGAAATATAATTCTGGATATCTGGCCGCCGTAGAGGCGCAGAAGGTCAAAG
ACAAAGAGGCAAGCGAACAACATGAGCAAGACAAAAAGACCATCGAGCAGGAAGCGCAAGGCCGCATTGAT
GCCGCGCGTGCTGATGCTTCCGCTGCTGCTGCTAAGTCTGGCAGGTTGCAGCAACAGCTCGCCACAATCAGAA
AGCAGCTCCTCGATTATTCCCGCACTGAGTCCATTGGCAATCCAGCCTCAAACACCGGAGTTTTGCTTGCCGAC
GTGCTCAGCAAATCTGTCGAAAGAAACAGACAACTGGCAGAATATGCTGACTCAGCAAGAGAAGCGGGATTG
ACTTGTCAGGCGCAATATAACTCTCTGAGGTCAGTTAATGCTAAATAGAGAGAAGCGCGAGGAGTATGACGG
AATGGCGGCCAATGTGGCGCTAGGCAGGGCCGGTTATCAACCAGCACGCAAAAGCCGGTACAGCAAGTCAGC
AAAAGATGAATATGGCCCTGCGCCTCAATTCCTGTAGCAATAAAAAAGCCCCATAAGGGGCTTGGCTTGGAA
AACTAAACAACGAAGCCGATATTTTCACCATCGAGTTTTGCGGCAATTTCAACCTCAATAATCATGGTGGCAA
TCTTTCCTCTCGATTGCGTGCTGGTGTCAGTTTTCACCACGCCGCCAACAATCCTCCCATCCTGGTCCACCACTT
GCAGGCGTCCGTCAATAATCTCCTGATGTAGCCAGATTTTTTCAGGGTCTTTCGCTGCTTGTACTGTCATTTTAA
ATCCTCATAGAGTGGTTTCATCTCATAGCCAAACATGGCTGCATTTTGGTGCTCAACACTACCAGCAAACGCC
AGATAGCGGCGACCACGATTGCTGGTGATGATGTAGGCCTTTGGTACTCTATTCTGCTTTACCGTCATTATTTA
CCAGCCTCCTCTGTCACTTTATCATTGGCGGCAGCGCGAAACAGCGTCGCGGTCAGATAAAATCCACCAAGCC
AGAAATAACCAAGCGCGAACATTACTGCTATTTCGCAAAATGTAGTCACAACCTGGTATTTATGATGAATCCA
TAGCGGCTTTTTGTAGCGAGCAACGGATTTATCAAGCAGGTCTTTGCTTGACAGTAAGCCAACTGATGCAATG
ATTGACGCAATCGACATGAACCAGAAAAAAGCCACAGCCATATTGTATGCATGCACATTCTGCATAAATAGGC
CGAAGTAAATCAGCGGCCACGTCAGGAATATATCCCATGCGATGTTTTTCAGGAGTCTTTTCATTTTGATAACC
TCGCCCAGCCTTTACCTTTCACGTTATAAACAAGCCCCTTCTTTTTCAGTGCCTGCATTCTTCTGTCAAGGATTG
TGATATTTTTAATTGGTGAAATGTCGAATACCTCAAACCAAATCGACCCGATATCTACAGGACGGATTGAATC
ATCAAGACGGCGCAGAATTGCATCGTCAAGTTCATCATAAGCACCCATCACTTTACCTCCCCGTTTAGTTCGTT
AACAATTAATGTTGCATAGCCAGCAATGTCTTTCCAGCTATCGTCGTATGTCGGGTCGCCATTCAGGATGCGAC
AGATTTTATGCTGAATCATGTCGAGTGCTTCTTTCTGGCTCGCTGTCAGGTTATTCCAGCCATCAACGTCGCGC
ATGGTGTCTTTCAGTGACTGCATGATTTCTGCACCGTCTTTGAATTTCCCGTAGCGTTTGCCTCGCTCGGTGATG
ATGGCTTCTGTGGAGTTGGCTTCTGTGGAGTTGGTGTGCCCATTCAAGTCATCATCAGTTACTGGCTCACGATG
AGCGATAACAATATCGCCAGACTTTTCGATATCCTTATCTCGCCCTGCATAATCCGCGCCGAGGTAGTGGATTT
TTCCACTGGTTGCTGATTTTACAGCAAGAACGGCATCTTCATGACCATTAAAATAATCAGCACTTCCCTTCAGG
TATTTGTATTTCATCACTCACCCCTCAATGTAACTTTGTTTTTCTCATCCACGCTGAAGTGTTCACGCACAAACG
CATACATTTCTTCAGCGCTCCATTCCCGAATTGCTACATAGCAGTGCGCGTAATATCTGACATCTCGCAGGCTT
AACGGCTGGCGCTTAGCGATAATCTCAGTCAATACTTCCAGTGGTTCTTTGCGTTGTCTCGCCATCTCTTCTCTC
CTCGTAAAATAGTATTGACTAATCTACGTCATCTAGTCAATACTTACTGGCGTAGATTGTATCATAGTTGCGAG
GAGTGGTGTGGAATGAAAAAGTGGCAGGAGGTGACAGAGGTTCATAAGCGCGATTGCCGGGAGACTCTGCAA
ATGCTTAATGTGCCAGAGTCAATAATTAAATCTATCGAGCAGCGCATTGACCTGGCTGCTATGGAGGCAGCCC

ATGAAGCCGAGGAAGCGCAAATGTTGTCATGGATGGGCAGAACTCTACCGGTCGTTTTACATCGTGGTAAAAG
TACCGATTGAAGATGATGACGGGTATCTGCACAACCATAGTCAGGTGCTCAAATATTATGGCGTTCACTACAA
AGTGCTGATGGAGAGAAAAAATGACTACTGATCAGGTGTACGAAAAAGAATTGCTGAACAATCTGGAAGAAC
TTGACCGCACTCGCGCATGGGTTGAAAGCGAATTGCACGAGGTTCGCAACCGCATGCAACGGCAGGTTAACC
GCGAAATTATCGAGTGGCGCGAAGGGCGCCCGCATTTCAGCAATATTGGTGAATGGGTGGCGAAATGAAACC
AATGATGAATGATGATGGGCTGCTGGAGTGTCCGTTTTGCAATAGCGATGATGTGTATCCTGACTCTGATTTAG
AGGGGAAGAAGCATTTTATTTGGTGTGGGAATTGCGGTTGTGGAACAGGAGTAAGACCTAACAAGTCATCAG
CTGTGCGCAAATGGAATACCCGCAACGGCCACCTCTATACCGCTGACGACTACAAACAAGATGCAGTGGAGC
GCGCAAATGGACTTTAAAACGCAAATACTCACCGTGATAGAGCGCTGCGGCGGTGCAACCAATGCCATGATA
CGCAAGCAGACTGGCATGACAAACCGAGCCAGCGTTACCGGCTATCTGATTGAACTGGAGAGCATGGGATTT
ATTATTAAAGAAGAGAGTGTCAGCTATGGCAGGCGCTGCTTTAAGTATTTCCTCAATCCCGGCAATACCGCGC
TCGATTTGGCAATTCAGACGTATCTTGAGGCGAATCCTGGGCGCAAAAGCAAGCAGATAGCAGAGGCTGTAG
GCGTCAACTACACCATCCTCAAGGCACGCATGCGCTATCTGGCAAGCATTGGTCAGGTTGACCGTGAAATGCT
TCCCGGCGGCGCATGGAAATATTACTGGCAGGAAATCATCCCGTTTGGAATGAGCCGTGACAGGATGATGTTT
GAAAAGCTGCTTGCTTGGGCGCGCCAGTCATGTGGGCTGTAAAGCATAAATCAGGAACCGTGATGTTTGTCAC
CAATTGTGAACGCACGGCCAATAATCGCAGAGAAATGGGGTGGATAGTGGAGGAAGTGAATGTGAGCAAACC
AAAAAGCGCATTAATTAAAATTACATGGCATGACGACACGGTTAGCCAGTGGCATGCAGATTCATTCAGAGA
GGGTGAGTACTCCATTGAAATGAATATTGGTGGATGCATCGTTTGCATCCCCTGGAGGTTATACAAAGATGGC
GAAGTTAAACATTTGGATGTGGAGTCGTAAATGAAACTACAACTTAACGAAATCATGGAAGCAACAATTAGC
GAACTGGATGACATTGATATGACGCTTGCCTTTGAGATTGAGGCTATTGAGCGCCAGCTTGCCGGTAATCATG
ATGCGGGCAGAGTATGGAGAGAGAAGGCCATGAAAGCGCGTGACCATATGAAACGCACCCGCGCACTGGTTC
GCACTCGACTTGATAAGCTCTACTACGGCGAAGAAAGAATGTTGCACGGCGCCATTCTGGCTGAAATCCGCAA
AACGATGCCTATTGGGAAATTCATGGATGCAGTAAACCGCGCAAAAATTAACTGCGGAATGCTAAATAAGAA
TAGTCCTCAATAAATCTTCATCCGTGGCTGTTACCTTGCATTCAGGAGGTAGCAGCCATGCCAATCATACTGAT
ATCATTCTTTGCTACTCTTTTCGCTTTTACCGCATCTCCGCTTTACCTTCTCGCGTCCGTTTCGTGGTGCATATTC
ATGGTGTGTTATAATCCGGGCATAAAGTAAGCGCGGAGAAAGGTCATGATTGTCAAAATTGGCGACAAGTGG
GTCGTTAAATCTAAGGATGGCTCGCACCAGTTTGGCGAGTACGACACCGAAGAGGCGGCGAAAAAGCGCCTT
GCTGAGGTGGAGGCATTCAAGCACATGAATAATAAATTACAGGTTAACATCCTGTACACCATCAACTCGGCCA
GCAACATCAGTGAAAAAATCATTGATGGCGATCCGCATTATGTCATCAAGAATGTTGTGCCGGTGGTAGATGA
TATTGTCATGAACGGCGGCCTGTACCCTGGCGATGAGATTAAAAAATCATTCCATGGTCTGGATGGAAAACCG
GCGCCATATGACCACCCGAAGATTGATGGCAAATATGTGTCAGCAAACATGACGCGAGCCGCCAATCAGTTC
AGCGTTGGTGCATGGATTGAGAACTCATCTCATGACGGCAGCAAAGCACTGGTAGACCTTTATATTAACAAGG
TGGTAGCTGAGCGCTCTGATAAAGGCAAAGAATTGCTGTCGCGCATTGACGGACTCAAAGTTAACAGCGCCG
ATGCTGAACCTGTTCAGGTGTCTACCGGTCTGTTGCTCAACCGTGAGCAGGCATCAGGAACTTCTAAGGGCAA
AAAATATTCCTGGATTGCCCGAAATATGGAGTGGGATCACCTCGCAATTCTTCCGCCTGGCATTCCTGGTGCTG
GCGGCCCTGCTGATGGTGTCGGCATCTTTGCTGCTAACGGCGAAGACATTGAGCGTGTTGTAGTCAACCTTGA
GGAATCGGCAATGCCAGACGAAAGTGCAAACAAAATCAAATGGTGGCAGCGTGCCATTAATCGCCTGACCGG
TAATCAGCTGTCATTCGCTGATATTACCGCACAACTCCACAATATCATCAAGGCCGATATGCAAGCCGACTCG
TGGCCCTATATCGTCGCTGTTTATGATAATTACTTCGGCGTTGAGATTGACGGCACCATTTATATGCAGTCCTA
CATCGTCCGCGAGGATATGGTAGAATTAGTCGGTGAACGGGTTAAGGCTGTTTATAAGACAGAGCTTGAACCG
GTAAAAACAACTCAAGGGGAAATCTCAATGACTAACGAGGAATTACAGGCTGTATTAGCCGATGCCCTCAAG
CCGGTTCAGGAATCGTTGACCGCAGTCAACCAGAAACTGGCCGATGTGGAGGCGCAAAACAAAACTCTGCGC
GACCAGCTGCAAGCCAATGCTGCGCAGGAAGAGACCGCAATGCGCGCCGCCATTATGGCTGAGCTGAAGCTG
CCGGAAGTTGCCGTTAATGCGCTGACTGGCGAAGCACTGCGTGAAACCTATGCACTCACCAGTAAAGCGGCTC
CGATTTCCGGCGGGTTCCAGCCGAACCGTGCCGAAGAAGATTTTGATATGGAGGCACCTGAATAATGGCTACT

ATCCGTTATGGCACCATCATCGGCGGCCCGGCCCGCAAAAACGACCCGCAGTTGCGCGAAGGTCTGATGAAC
GTCGCCCTGCAACCTGGCGCACTGGTCGACTTCAACTCCTCTGACAAAATCATCGCGCATGCGACTGCTGGCG
GTCATGGTTTCCCTTACGTCCTGCAACACAACTATGTTGGTGGCGGTGACGTGAGCGAAGCTGTACCGGCGAA
TGCTACCGGCATGGCAGTACAGTGCGAATTTGGCGTAACGTATCACGCTCTGGTTGCGGCATCATCCGCGCTG
GTAAAAGGTACTCCGCTGGCAAGCAATGGCTCCGGCGCGTTAAAAGTGGCTGGCACAGGTGATAAAATCCTG
TTCTATGCGTATGAAGCCTACACCGTAGCATCTGATGGCGCTGAACTCGTTGCAGTTCGTCGTGCTGGCAATGC
TGCAATGCC

The second embodiment of the invention refers to the bacteriophage/s of the invention for use as a medicament, preferably for use in the prevention or treatment of a bacterial infection caused by *K. pneumoniae.*

The third embodiment of the invention refers to a pharmaceutical composition comprising the bacteriophage/s of the invention, and, optionally, pharmaceutically acceptable excipients or carriers.

The fourth embodiment of the present invention refers to the pharmaceutical composition comprising the bacteriophage/s of the invention, for use as a medicament, preferably for use in the prevention or treatment of a bacterial infection caused by *K. pneumoniae.*

Alternatively, the present invention refers to a method for preventing or treating a bacterial infection caused by *K. pneumoniaewhich* comprises the administration of a therapeutically effective dose or amount of the bacteriophage/s of the invention or a pharmaceutical composition comprising thereof.

In a preferred embodiment the bacteriophages of the invention are administered along with one or more antibiotics, wherein the antibiotics are administered before, after or the same time than the bacteriophages of the invention. The antibiotics to be administered in combination with the bacteriophage/s of the invention are preferably selected from: Beta-lactam antibiotics such as penicillin or cephalosporin, aminoglycoside, carbapenems such as imipenem or meropenem, quinolones, or combinations thereof, preferably polymyxin B and/or colistin.

The last embodiment of the present invention refers to a method of detecting the presence of *K. pneumoniae*in an *in vitro* sample, for instance obtained from a subject, wherein the subject is preferably a patient, comprising contacting said sample with the bacteriophage/s of the invention and determining whether said bacteriophage/s kills bacteria in said sample.

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of' is meant including, and limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.

### Description of the figures

**Figure 1****.** pH stability of bacteriophage F12A and F13. Incubation for 1h at pH of 1, 4.5, 7.4, and 8. The bars represent the median and the interquartile range. Discontinued line denotes limit detection of quantification (10 PFU/ml).
**Figure 2****.** Phage temperature stability. Incubation for 1 h, 24 h and 168 h at temperatures of - 80°C, -20°C, 4°C, 21°C, 37°C and 60°C. The bars represent the median and the interquartile range. Discontinued line denotes limit detection of quantification (10 PFU/ml).
**Figure 3****.** Bacteriophage Inhibition Assay. Inhibition of KP5 standard clinical strain after treatment with individual phages at MOI (10, 1 and 0.1). Grey bars represent the interquartile range.
**Figure 4****.**F12A and F13 effect on KP5 clinical strain biofilm growth in both planktonic state **(a, c)** and biofilm form **(c, d)** at 6h **(a-b)** and 24h **(c-d).** The bars represent the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

### Detailed description of the invention

The present invention is illustrated by means of the Examples set below without the intention of limiting its scope of protection.

### Example 1. Material and Methods

### Example 1.1. Bacterial strains and growth conditions

*Klebsiella pneumoniae* clinical strain KP5 and other clinical isolates were obtained from patients (n=58) and donated by the Department of Microbiology, Hospital Universitario Fundación Jimenez Diaz (HUFJD). The presence *of K. pneumoniae* on clinical isolates was confirmed using MALDI-TOF (Bruker, VIC, Australia). The *K. pneumoniae* clinical isolates were stored in Difco^{™} skimmed milk (East Rutherford, NJ, United States) at -80 °C. The clinical isolates were plated from frozen skimmed milk stocks onto Mueller Hinton E agar 90 mm plates and broth cultures were grown in tryptic soy broth (TSB) (BioMerieux, France). Agar plates and broth cultures were incubated at 37 °C.

### Example 1.2. Bacteriophage Isolation

The phages isolated against *K. pneumoniae* strain KP5 were obtained from HUFJD sewage (Madrid, Spain). Samples of 50 ml were centrifuged at 4000 x g for 10 min to remove cellular debris and faecal matter. The supernatant was filtered using 0.45 µm and 0.22 µm filters to remove the bacteria and debris. An amount of 100 µl of filtered solution was combined with 100 µl of the KP5 overnight culture and 3 mL of agar 0.2% (W/V) and plated on MHE plates via the double-layer agar method. Following overnight incubation, the formation of plaques confirmed the presence of phage. Individual plaques with different morphology were picked using pipette tips and placed into 1.5 ml Eppendorf tubes (Eppendorf, Hamburg, Germany) containing sodium magnesium buffer (SM; 100 mM sodium chloride (Panreac Química, Illinois, United States); 10 mM magnesium sulfate (Thermo Fisher Scientific, Waltham, MA, USA); 10 mM calcium chloride (Thermo Fisher Scientific, Waltham, MA, USA);50 mM Tris HCl (Sigma-Aldrich, Castle Hill, NSW, Australia), pH 7.5), and vortexed vigorously before storage at 4°C.

### Example 1.3. Bacteriophage propagation

A two-step propagation was applied to amplify and purify the isolated phage. For small-scale amplification of phage, 100 µl of KP5 overnight culture was added into 10 ml TSB and incubated at 37°C with 200 rpm shaking for 1.5 h to reach exponential growth. An amount of 100 µl of phage in SM buffer, 150 µl of MgSO₄ and CaCl₂ (200 mM) was added into the coculture and incubated for 4-5h at 37°C with 200 rpm shaking. The supernatant containing phage was harvested after centrifugation (4000 x g, 15 min) and filtration (0.45 µm and 0.22 µm syringe filter). Phage titration was performed. The small-scale amplification was applied according to the prior art.

For large-scale amplification of phage, 500 µl of the KP5 overnight culture was incubated with 50 ml of TSB for 20 min. After incubation, 100 µl of phage and 2.5 ml of MgSO₄ and CaCl₂ were then added into the coculture and incubated overnight at 37°C with 200 rpm shaking. The phage lysis was harvested as described above. The large-scale amplification was applied according to the prior art.

### Example 1.4. Bacteriophage titration

The phage titer was determined via a double-layer agar method. Briefly, 100 µl of the KP5 overnight broth culture was added to 3 ml of agar (0.2% (W/V)) and overlaid onto a MHE plate. Phages were serially diluted in SM buffer. The plates were incubated at 37°C overnight. The phage titer was counted and calculated.

### Example 1.5. Bacteriophage Stability testing

The stability *of K. pneumoniae* phage was tested against a wide pH (1-8) and temperature (-80°C to 60°C) range using a working stock in TSB broth with an initial phage titer of around 10⁸ PFU·ml⁻¹. Briefly, 100 µl working stock of each phage was suspended in 1 ml SM buffer previously adjusted with 1M NaOH or HCl (Sigma-Aldrich, Castle Hill, NSW, Australia) to yield a pH range of 1-8. The samples were incubated at room temperature for 1 h. The pH stability testing was studied according to the methods described in the prior art. For thermal stability, 100 µl of working stock phage was suspended in 1 ml SM buffer and incubated at - 80°C, -20°C, 4°C, 21°C, 37°C, and 60°C for 1, 24 and 168 h (1 week). The phage stability for each experiment was determined by measuring the phage titration. The thermal stability testing was studied according to the methods described in the prior art.

### Example 1.6. Bacteriophage Host Range Analysis

Host range of F13 phage was determined against the different clinical *Klebsiella pneumoniae* strains obtained from Hospital Universitario Fundación Jiménez Diaz (HUFJD) using spot testing. Briefly, 3 µl of the purified phage suspension (10⁸ PFU/ml) was spotted onto the surface of double-layer agar plate previously inoculated with the tested strains. After adsorption of phage suspension, plates were incubated overnight at 37°C. Host range was determined by plaque formation. Three replicates were tested for each bacterial strain. The host range test was applied according to methodology described in the prior art.

### Example 1.7. Bacteriophage Inhibition assays

The infectivity profile of the *K. pneumoniae* bacteriophages was assessed at multiplicity of infection (MOI) 0.1, 1 and 10 using inhibition assays in liquid. Briefly, the McFarland inoculation standards (0.5±0.02) of each clinical isolate (~10⁸ UFC·ml⁻¹) were prepared and the required volume of phage stock solution (10⁸PFU·ml⁻¹) was added to the corresponding suspension to achieve MOI 0.1, 1 and 10. The samples were incubated at 37°C with shaking orbital amplitude of 5 mm. Every 5min for 15 h the OD₅₉₅ₙₘ value was measured.

### Example 1.8. Phage effect on K. pneumoniae biofilm

The isolated phage effect on *K. pneumoniae* biofilm was determined using a modified methodology previously described in the prior art. Briefly, biofilm formation on the bottom of a MicroWell 96-well flat-bottom plate (Thermo Fisher Scientific, Waltham, MA, USA) was induced by inoculating 100 µL of MHB containing ~10⁷ CFU·ml⁻¹ of bacteria per well and the plate was incubated at 37°C and 5% CO₂ for at least 18 h. The next day, the wells were aspirated, and biofilm formation was treated with bacteriophages at different concentrations (10⁷,10⁶, 10⁵ and 10⁴ pfu/mL) in order to study the inhibition of biofilm by phages. This study was done at two different incubation times with the treatment: 6h and 24h. After 6h and 24h of treatment with phages, 100 µl from the biofilm formation were transferred to a new 96-well untreated microtiter plate in order to separate planktonic bacteria from biofilm. From the biofilm plate, wells were aspirated, and each well was washed one time with 200 µl of physiological serum (0.9% W/V). Planktonic bacterial viability was determined by addition of 20 µl of 5 mg/mL of 3-(4,5-dimethylthiazol-2-yl)-2,5- diphenyltetrazolium bromide (MTT) (Sigma Aldrich, Merck, Darmstadt, Germany) (2) while biofilm viability was determined by adding 1mL of MTT to a commercial 9mL TSB tube and incubating for 1 h at 37°C and 5% CO₂. The absorbance was measured at 570 nm for MTT using a microplate reader (TECAN). This experiment was performed in duplicate.

### Example 1.9. Phage-antibiotic checkerboard

Antibacterial synergistic activity for F12A and F13 phages was assigned by modified broth microdilution method in flat-bottomed 96 multi-well microplates (Thermo-Fischer). Experimental conditions were designed for antibiotic-phage mix and were tested for colistin, polymyxin B, doripenem, ciprofloxacin and bacterial control at the final volume of 200 µl. Briefly, the culture was allowed to grow until an OD₆₀₀ of 0.44 reached 4×10⁷ CFU. Then 50 µl of antibiotic at different concentrations was added to each well containing 50 µl of Mueller Hinton Broth II (Millipore, Sigma Aldrich, Merck) supplemented with 10 mM CaCl₂ and 10 mM MgSO₄. In addition, 50 µl of the phage dilutions (10⁷ PFU) were added to the suspension and 100 µl of bacterial suspension was added to the mix wells. Plates were incubated at 37°C overnight and FICI was calculated according to bacterial growth.

### Example 1.10. Statistical Analysis

Data distribution was evaluated using Shapiro-Wilk or Kolgomorov-Smirnov statistics. Descriptive statistics are cited as median and interquartile range (non-normal distribution) for each variable that were calculated. Non-parametric Mann-Whitney test considering equality of variances was used to compare two groups and non-parametric Kruskal-Wallis's test was used to compared more than two groups. To determine the effect of temperature on the phage viability data was analyzed using linear regression. Bacteriophage inhibition of bacterial biofilm was analyzed by Dunn's pairwise test with a Benjamini-Hochberg's procedure. The possible relation between the bacterial biofilm or planktonic concentration and the concentration of phage was determined by using Spearman rank correlation coefficient. Significance level was established at α=0.05.

All statistical analysis was performed using R (R Core Team, 2017) with R commander, except for linear regressions that were carried out using GraphPad Prism v.8 (GraphPad Prism, version 8.0.1 (86); Windows Version by Software MacKiev ^{©} 2020-2018 GraphPad Software, LLC.; San Diego, CA, USA) and STATA statistical software, release 11 (StataCorp, 2009, StataCorp LP., Texas, USA).

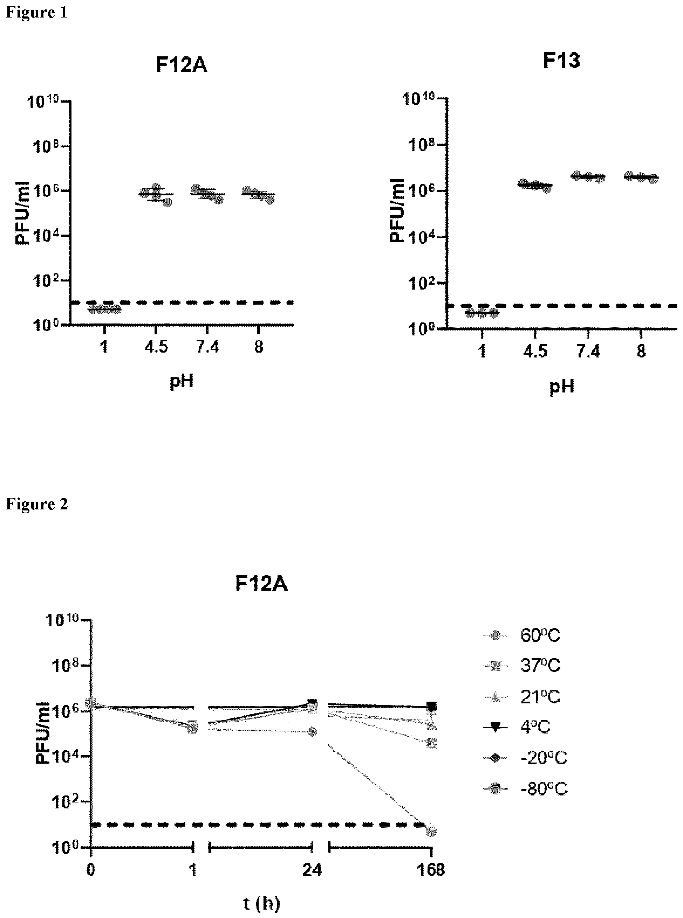

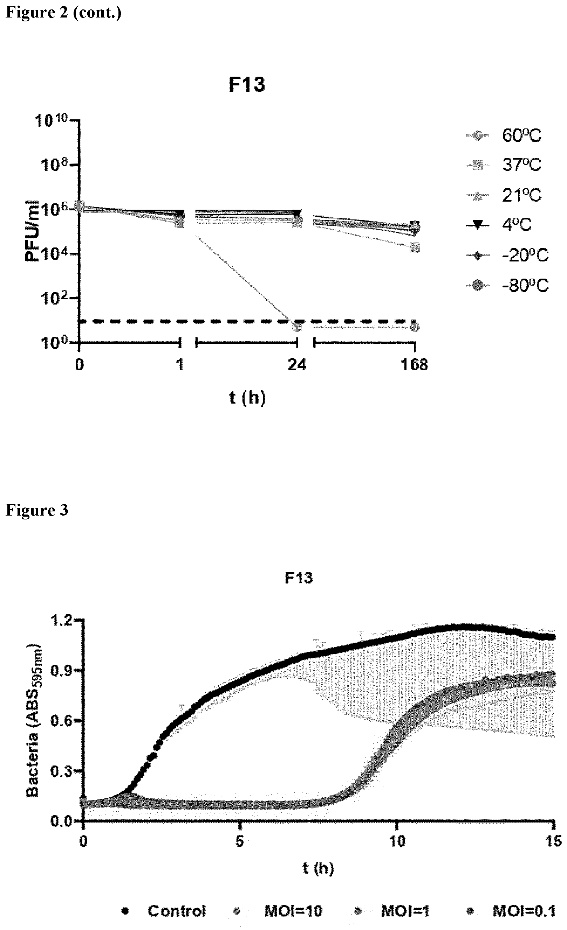

Departing from these materials and methods, and the common general knowledge, the present invention could be reproduced by the person skilled in the art departing from the genetic material of the bacteriophages (for instance SEQ ID NO: 1). For instance, the reference [Jiang S, Tang Y, Xiang L, Zhu X, Cai Z, Li L, Chen Y, Chen P, Feng Y, Lin X, Li G, SharifJ, Dai J. Efficient de novo assembly and modification of large DNA fragments. Sci China Life Sci. 2021 Dec 16. doi: 10.1007/s11427-021-2029-0. Epub ahead of print. PMID: 34939159*]* explains how to synthesise de novo long DNA from sequences. On the other hand, the reference [Diana P. Pires et al., 2021.Designing P. aeruginosa synthetic phages with reduced genomes. Scientific Reports | (2021) 11:2164 | https://doi.org/10.1038/s41598-021-81580-2] explains how phages can be synthesized and a general scheme is provided therein (see Figure 2 of this paper).

### Example 2. Results

### Example 2.1. Bacteriophage Stability testing

Phages were tested against a wide pH range (pH 1, 4.5, 7.4 and 8) after 1h incubation period, to establish their stability under neutral, acidic and alkaline conditions (Fig. 1). After 1 h incubation at pH 1, no plaques were detected in 100 ul of sample, which suggested that the bacteriophage is unstable at this pH (p-value<0.05). The rest of pH tested did not produce logarithmic reductions after 1 hour of treatment in F12A and F13.

Phages were also tested after treatments with different temperatures: -80°C, -20°C, 4°C, 21°C and 37°C and 60°C **(****Fig.2****).** F12A and F13 were stable at -80°C, -20°C, 4°C and 21°C after seven days of exposition. A certain reduction of phage viability was observed after seven days at 37°C. The main reduction of viability was observed at 60°C after 24 hours.

### Example 2.2. Bacteriophage Host Range Analysis

The host range against 58 *K. pneumoniae* clinical isolates for phages F7, F8, F12A, F12B, F13 and F14 showed 10 susceptible (+) strains including a pan-resistant strain **(Table 1)**. All tested strains were multi-drug resistant (MDR) *K. pneumoniae* strains including KP5 strain.

**Table 1**

| **Strain** | **Resistance** | **I/C** | **Origin** | **Susceptible to 6 phages** |
|---|---|---|---|---|
| **KP5** | **MDR** | **I** | **Unknown** | **+** |
| **KP21** | **MDR** | **I** | **Urine** | **+** |
| **KP22** | **MDR** | **I** | **Urine** | **+** |
| **KP29** | **MDR** | **I** | **Urine** | **+** |
| **KP32** | **MDR** | **I** | **Urine** | **+** |
| **KP52** | **MDR** | **I** | **Urine** | **+** |
| **KP74** | **MDR** | **I** | **Urine** | **+** |
| **A7** | **MDR** | **I** | **Wound** | **+** |
| **A29** | **MDR** | **I** | **Urine** | **+** |
| **KP4940164** | **MDR** | **I** | **Bronchial** | **+** |

| | | | | |
|---|---|---|---|---|
| *Summary of phages Host Range. K**.** pneumoniae strains susceptible (*+*) to 6 phages against K.pneumoniae based on the degree of lysis on the bacterial lawn by counting the spots. I: Infection; C: colonization.* | | | | |

### Example 2.3. Bacteriophage Inhibition assays

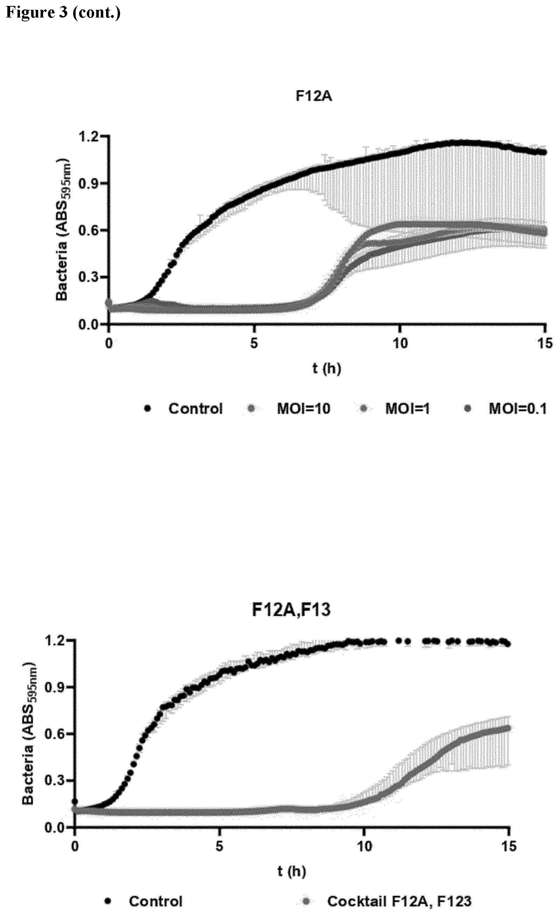

The phages planktonic infective properties were determined through inhibition assays. The phage inhibition of KP5 clinical isolate was assessed for 15 h when infected at MOI 10, 1 and 0.1 for each phage **(****Fig. 3****).**

### Example 2.4. Minimum Inhibitory Biofilm Concentration

The concentration of KP5 in planktonic state decreased by 35% at 10¹⁰ PFU/ml concentration of bacteriophage F12A (p-value<0.05) after 6h of treatment **(Fig. F12A 4a),** and was reduced by 32% at 10⁸ PFU/mL after 24h of treatment **(Fig. F12A 4b).** Biofilm formation was reduced by 28% at 6 h and 10¹⁰ PFU/mL of F12A **(Fig. F12A 4c).**

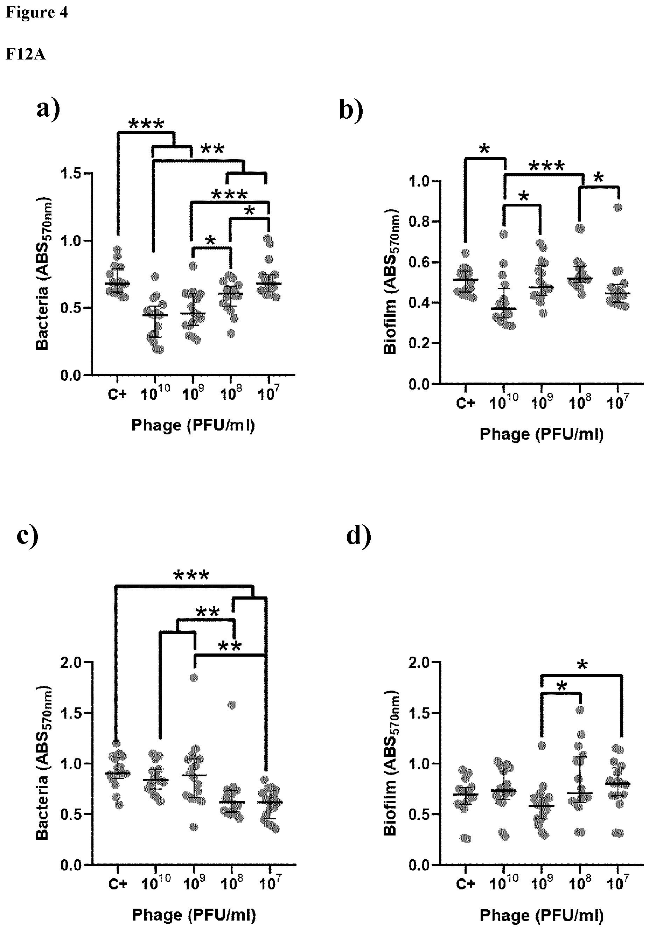

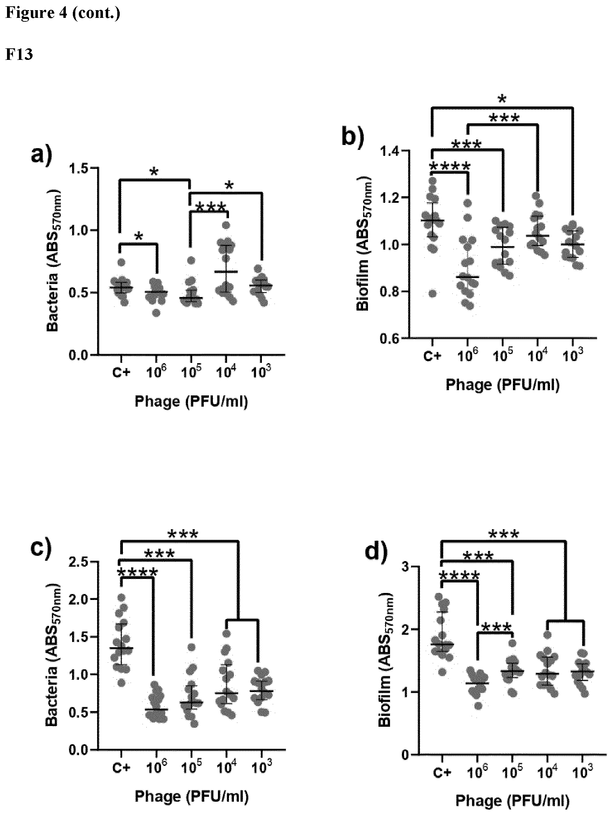

The concentration of KP5 bacteria in planktonic state and biofilm formation showed a significant decrease by 15% **(Fig. F13 4a)** and 22% **(Fig. F13 4b),** respectively, with 10⁶ PFU/ml of F13 (p-value<0.05) after 6 h of treatment. The concentration of planktonic bacteria was reduced by 60% at 10⁶ PFU/ml after 24h of treatment **(****Fig. 4c****)** and biofilm formation was reduced 35% after 24 h of treatment with 10⁶ PFU/ml of F13 **(****Fig. 4d****).**

### Example 2.5. Phage-antibiotic interaction

Phage-antibiotic checkerboard assays showed synergy between F13 and polymyxin B (FIC index 0.135) and an additive effect with colistin (FIC index 0.501) **(Table 2).** Polymyxin B was very effective against KP5, 8 µg/ml of polymyxin B was able to lyse the cells in combination with F13 at phage titer of 10⁵ and 10⁶ PFU/ml demonstrating synergistic effect. F12A was very effective in combination with polymyxin B (FIC index 0.26), doripenem (FIC index 0.251), colistin (FIC index 0.015) and ciprofloxacin (FIC index 0.35) at phage titer of 10⁸ PFU/ml.

**Table 2**

| **Antibiotic** | **FIC index** | | |
|---|---|---|---|
| | **F12A** | **F13** | **Effect** |
| **Polymyxin B** | **0.26** | **0.135** | **Synergy / Synergy** |
| **Colistin** | **0.015** | **0.501** | **Synergy / Addition** |
| **Ciprofloxacin** | **0.35** | | **Synergy** |
| **Doripenem** | **0.251** | | **Synergy** |

| | | | |
|---|---|---|---|
| *Summary of synergistic effect between F12A, F13 and antibiotics against KP5 K**.** pneumoniae strain.* | | | |

## Claims

1. Bacteriophage, **characterized by** their specificity for *Klebsiella pneumoniae* strains, selected from a bacteriophage deposited at the German Collection of Microorganisms and Cell Cultures GmbH (DSMZ) with deposit number DSM34222; a bacteriophage **characterized by** comprising a nucleic acid consisting of SEQ ID NO: 1; or a bacteriophage comprising a nucleic acid having at least 95% of identity with the nucleic acid of the bacteriophage DSM 34222 or the SEQ ID NO: 1.

2. Bacteriophage, according to claim 1, deposited at the German Collection of Microorganisms and Cell Cultures GmbH (DSMZ) with deposit number DSM34222.

3. Bacteriophage, according to any of the claims 1 or 2, for use as a medicament.

4. Bacteriophage for use, according to claim 3, in the prevention or treatment of a bacterial infection caused by *Klebsiella pneumoniae.*

5. Bacteriophage for use, according to any of the claims 3 or 4, in combination with an antibiotic, preferably selected from: polymyxin B and/or colistin.

6. Pharmaceutical composition comprising a bacteriophage according to claims 1 or 2 and, optionally, pharmaceutically acceptable excipients or carriers.

7. Pharmaceutical composition, according to claim 6, further comprising an antibiotic preferably selected from: polymyxin B and/or colistin.

8. Pharmaceutical composition according to claims 6 or 7 for use as a medicament.

9. Pharmaceutical composition for use, according to claim 8, in the prevention or treatment of a bacterial infection caused by *Klebsiella pneumoniae.*

10. A method of detecting the presence of *Klebsiella pneumoniae*in an *in vitro* sample, comprising contacting said sample with a bacteriophage according to claims1 or 2, and determining whether said bacteriophages kill bacteria in said sample.
